(19) **Europäisches Patentamt / European Patent Office / Office européen des brevets**

(11) **EP 4 491 668 A1**

(12) # EUROPEAN PATENT APPLICATION
published in accordance with Art. 153(4) EPC

(43) Date of publication:
**15.01.2025 Bulletin 2025/03**

(21) Application number: **23766711.8**

(22) Date of filing: **02.03.2023**

(51) International Patent Classification (IPC):
**C08L 53/02** (2006.01)

(52) Cooperative Patent Classification (CPC):
**C08L 53/02**

(86) International application number:
**PCT/JP2023/007889**

(87) International publication number:
**WO 2023/171535 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.03.2022 JP 2022037275**

(71) Applicant: **Zeon Corporation**
**Tokyo 100-8246 (JP)**

(72) Inventor: **ISHII, Yuta**
**Tokyo 100-8246 (JP)**

(74) Representative: **Kraus & Lederer PartGmbB**
**Thomas-Wimmer-Ring 15**
**80539 München (DE)**

(54) **THERMOPLASTIC ELASTOMER**

(57) Provided is a thermoplastic elastomer, wherein the storage modulus at 23°C is more than 0.3 MPa; the loss tangent ($\tan\delta$) at 23°C is less than 0.20; when the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5; and when the film is stretched in the MD direction, the film does not have a yield point at an elongation rate of 200% or less.

## Description

TECHNICAL FIELD

The present invention relates to a thermoplastic elastomer, and more particularly, to a thermoplastic elastomer which can provide a film-shaped elastic body producing a high stress and having a high stretchability and a large width.

BACKGROUND ART

Since an aromatic vinyl-conjugated diene-aromatic vinyl block copolymer is particularly rich in elasticity and flexible among thermoplastic elastomers, its application as a material for a stretchable film used in hygienic products such as paper diapers and sanitary products has become one of its representative applications.

In hygienic products such as paper diapers and sanitary products, elastic films are used in various parts because the following property and fit to the movement of the wearer are required. For example, Patent Document 1 discloses a composition containing a specific block copolymer as a composition for providing an elastic body which is to be used as hygienic products such as paper diapers. According to Patent Document 1, an elastic body having a small variation in film thickness and a small permanent tension set can be obtained.

RELATED ART DOCUMENT

PATENT DOCUMENT

Patent Document 1: WO 2020/045496

SUMMARY OF THE INVENTION

PROBLEMS TO BE SOLVED BY THE INVENTION

On the other hand, an elastic body used in stretchable member applications such as a shorts-type diaper, which is a kind of paper diaper, is required not only to have a small permanent tension set, but also to produce a high stress. Although Patent Document 1 discloses the elastic body having a small permanent tension set, Patent Document 1 is not focused on achieving a high stress and does not disclose any specific means for achieving a high stress.

In addition, as a production process for efficiently producing a stretchable member for hygienic products such as a shorts-type diaper, a process has been studied to obtain a stretchable member (a stretchable composite sheet) by heating and melting an elastic resin material for constituting the stretchable member, discharging the molten elastic resin material into a film or line shape, then stretching the discharged material in the MD direction to produce a stretched elastic resin material, and laminating and bonding the stretched material onto a substrate sheet. When the composition disclosed in Patent Document 1 is used in this process, the discharged film excessively shrinks in width, and when the sheet is stretched in the MD direction, the stretched film excessively shrinks in width. Thus, the composition does not allow production of a wide stretchable member with efficiency.

The present invention has been made in consideration of such circumstances. An object of the present invention is to provide a thermoplastic elastomer which can provide a film-shaped elastic body producing a high stress and having a small permanent tension set and a large width.

MEANS FOR SOLVING THE PROBLEMS

The present inventors, who have conducted research to achieve the above object, have found that the above object can be achieved when a thermoplastic elastomer is made such that the thermoplastic elastomer has a storage modulus and a loss tangent ($\tan\delta$) within specific defined ranges, and when a film is formed from the thermoplastic elastomer by melt extrusion, the film has specific properties. These findings have led to completion of the present invention.

That is, the present invention provides a thermoplastic elastomer,

wherein the storage modulus at 23°C is more than 0.3 MPa;
the loss tangent ($\tan\delta$) at 23°C is 0.20 or less;
when the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5; and

when the film is stretched in the MD direction, the film does not have a yield point at an elongation rate of 200% or less.

The thermoplastic elastomer according to the present invention preferably contains an aromatic vinyl block copolymer.

In the thermoplastic elastomer according to the present invention, the aromatic vinyl block copolymer is preferably an aromatic vinyl-conjugated diene block copolymer.

The elastomer according to the present invention preferably contains the aromatic vinyl block copolymer in a proportion of more than 50% by weight, and a polyolefin elastomer in a proportion of more than 1% by weight and less than 50% by weight.

In the thermoplastic elastomer according to the present invention, the polyolefin elastomer preferably contains an ethylene unit.

The thermoplastic elastomer according to the present invention is preferably for use in a stretchable member which is stretched and contracted in the MD direction.

The thermoplastic elastomer according to the present invention is preferably used as a stretchable member by extruded into a film shape by melt extrusion, and successively laminated and welded onto a substrate sheet.

The present invention provides an elastic body obtained by using the above thermoplastic elastomer.

In addition, the present invention provides a film obtained by using the above thermoplastic elastomer.

The elastic body or film according to the present invention can be used in applications where the elastic body or film is stretched and contracted in the CD direction, but preferably is used in applications where the elastic body or film is stretched and contracted in the MD direction.

In addition, the present invention provides a porous film obtained by using the above thermoplastic elastomer.

Furthermore, the present invention provides a stretchable member obtained by using the above thermoplastic elastomer.

EFFECTS OF THE INVENTION

The present invention provides a thermoplastic elastomer which can provide a film-shaped elastic body producing a high stress and having a small permanent tension set and a large width.

BRIEF DESCRIPTION OF DRAWING

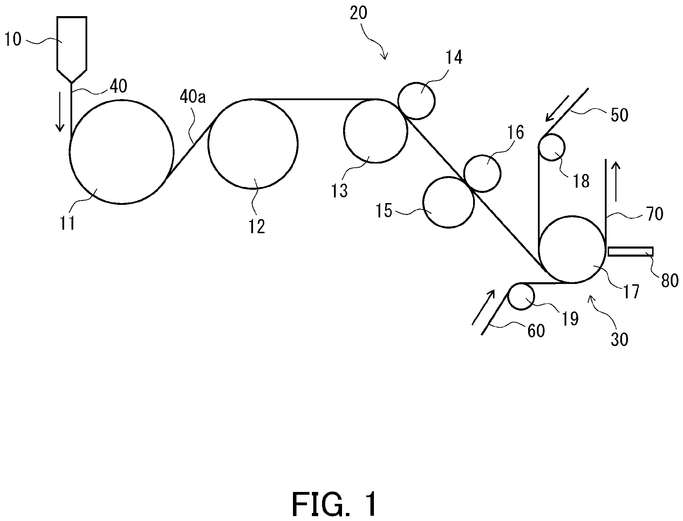

Fig. 1 shows a schematic illustration of a production apparatus for producing a stretchable laminate according to one embodiment of the present invention.

DESCRIPTION OF EMBODIMENTS

The thermoplastic elastomer according to the present invention satisfies all of the following (1) to (4):

(1) The storage modulus at 23°C is more than 0.3 MPa.
(2) The loss tangent (tan$\delta$) at 23°C is 0.20 or less.
(3) When the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5.
(4) When the thermoplastic elastomer is extruded into a film shape by melt extrusion using a melt extrusion apparatus, and cooled and solidified to form a film and the film is stretched in the MD direction, the film does not have a yield point at an elongation rate of 200% or less.

The MD direction of a film is the melt flow direction when the thermoplastic elastomer is extruded into a film shape by melt extrusion using a melt extrusion apparatus, and cooled and solidified to form the film, and the TD direction of the film is the melt flow transverse direction when the thermoplastic elastomer is extruded into a film shape by melt extrusion using a melt extrusion apparatus, and cooled and solidified to form the film.

As a result of extensive studies on a thermoplastic elastomer which can provide a film-shaped elastic body producing a high stress and having a small permanent tension set and a large width, the present inventors have found that a thermoplastic elastomer which satisfies all of the above-described (1) to (4) can provide a film-shaped elastic body producing a high stress and having a small permanent tension set and a large width, thereby having completed the present invention.

In the thermoplastic elastomer according to the present invention, the storage modulus at 23°C is more than 0.3 MPa, preferably more than 0.3 MPa and less than 8 MPa, more preferably 0.4 to 5 MPa, still more preferably 0.45 to 3 MPa, still more preferably 0.5 to 2.5 MPa, even more preferably 0.55 to 2.1 MPa, particularly preferably 0.6 to 1.8 MPa. When the storage modulus at 23°C is too small, the stress becomes low. The storage modulus at 23°C can be measured by a method described in Examples described below.

In the thermoplastic elastomer according to the present invention, the loss tangent ($\tan\delta$) at 23°C is 0.20 or less, preferably less than 0.16, more preferably less than 0.14, most preferably less than 0.12. The lower limit of the the loss tangent ($\tan\delta$) at 23°C is not particularly limited, but is usually 0.01 or more. When the loss tangent ($\tan\delta$) at 23°C is too high, the permanent tension set becomes large. The loss tangent ($\tan\delta$) at 23°C can be measured by a method described in Examples described below.

In the thermoplastic elastomer according to the present invention, when the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5, preferably 1.0 to 2.0, more preferably 1.05 to 1.9, most preferably 1.1 to 1.5. When the ratio ($M_{MD}/M_{TD}$) is too large, when the thermoplastic elastomer is heated and melted, and then extruded into a film shape, the extruded film excessively shrinks in width (the width of the resulting film becomes too small relative to the width when the thermoplastic elastomer is extruded), and when the film is stretched in the MD direction, the stretched film excessively shrinks in width (the width of the film becomes too small relative to the width of the film before it is stretched in the MD direction). Thus, a film-shaped elastic body having a large width cannot be obtained, and efficient production of a film-shaped elastic body having a large width becomes impossible. In particular, when a film stretched in the MD direction excessively shrinks in width and such a stretched film is laminated onto a substrate sheet to form a laminate, the width of the laminate becomes small.

As long as the reduction in the width of film is acceptable, by controlling such that the ratio ($M_{MD}/M_{TD}$) exceeds 1.0, a film-shaped elastic body exhibiting a further high stress can be obtained. The ratio ($M_{MD}/M_{TD}$) can be measured by a method described in Examples described below.

In addition, in the thermoplastic elastomer according to the present invention, when the thermoplastic elastomer is extruded into a film shape by melt extrusion using a melt extrusion apparatus, and cooled and solidified to form a film and the film is stretched in the MD direction, the film does not have a yield point at an elongation rate of 200% or less. When the film has a yield point at an elongation rate of 200% or less, the permanent tension set becomes large. Whether or not a film has a yield point at an elongation rate of 200% or less can be measured by a method described in Examples described below.

The thermoplastic elastomer according to the present invention is not particularly limited as long as the thermoplastic elastomer satisfies all of the (1) to (4) above, but preferably contains an aromatic vinyl block copolymer, more preferably contains an aromatic vinyl block copolymer as the main component (e.g., contains the aromatic vinyl block copolymer in a proportion of more than 50% by weight in the whole thermoplastic elastomer), still more preferably contains an aromatic vinyl block copolymer in a proportion of more than 75% by weight. Among the aromatic vinyl block copolymers, an aromatic vinyl-conjugated diene block copolymer is preferred.

(Aromatic vinyl block copolymer composition)

In the present invention, preferred examples of thermoplastic elastomers that suitably satisfy all of the (1) to (4) above include the following aromatic vinyl block copolymer composition.

That is, preferred examples of such thermoplastic elastomers include an aromatic vinyl block copolymer composition comprising:

a block copolymer A represented by the general formula (A): Ar1$^a$-D$^a$-Ar2$^a$,
a block copolymer B represented by the general formula (B): Ar1$^b$-D$^b$-Ar2$^b$, and
at least one of a block copolymer C represented by the general formula (C): Ar1$^c$-D$^c$-Ar2$^c$ and a block copolymer D represented by the general formula (D): (Ar$^d$-D$^d$)$_n$-X.

The block copolymer A represented by the general formula (A), Ar1$^a$-D$^a$-Ar2$^a$, is preferably a block copolymer described below.

In the formula, Ar1$^a$ represents an aromatic vinyl polymer block having a weight average molecular weight of 7,000 to 17,000, D$^a$ represents a conjugated diene polymer block having a weight average molecular weight of 50,000 to 200,000, and Ar2$^a$ represents an aromatic vinyl polymer block having a weight average molecular weight of more than 20,000 and 300,000 or less.

The weight average molecular weight of the block copolymer A (that is, the weight average molecular weight of the entire block copolymer A) is 80,000 to 400,000, the molecular weight distribution (Mw/Mn) of the block copolymer A (that is, the molecular weight distribution (Mw/Mn) of the entire block copolymer A) is 1.0 to 1.2, the content of the aromatic vinyl monomer unit of the block copolymer A is 39 to 85% by weight with respect to the total monomer units constituting the block copolymer A, and the cis/trans ratio of the conjugated diene monomer units constituting the conjugated diene polymer block ($D^a$) is 1.5 to 4 (i.e., cis/trans = 1.5 to 4).

The block copolymer B represented by the general formula (B), Ar1$^b$-D$^b$-Ar2$^b$, is preferably a block copolymer described below.

In the formula, Ar1$^b$ represents an aromatic vinyl polymer block having a weight average molecular weight of 7,000 to 17,000, D$^d$ represents a conjugated diene polymer block having a weight average molecular weight of 50,000 to 200,000, and Ar2$^b$ represents an aromatic vinyl polymer block having a weight average molecular weight of 7,000 to 17,000.

The weight average molecular weight of the block copolymer B (that is, the weight average molecular weight of the entire block copolymer B) is 75,000 to 300,000, the molecular weight distribution (Mw/Mn) of the block copolymer B (that is, the molecular weight distribution (Mw/Mn) of the entire block copolymer B) is 1.0 to 1.2, the content of the aromatic vinyl monomer unit of the block copolymer B is 21 to 30% by weight with respect to the total monomer units constituting the block copolymer B, and the cis/trans ratio of the conjugated diene monomer units constituting the conjugated diene polymer block ($D^b$) is 1.5 to 4 (i.e., cis/trans = 1.5 to 4).

The block copolymer C represented by the general formula (C), Ar1$^c$-D$^c$-Ar2$^c$, is preferably a block copolymer described below.

In the formula, Ar1$^c$ represents an aromatic vinyl polymer block having a weight average molecular weight of 7,000 to 17,000, D$^c$ represents a conjugated diene polymer block having a weight average molecular weight of 80,000 to 250,000, and Ar2$^c$ represents an aromatic vinyl polymer block having a weight average molecular weight of 7,000 to 17,000.

The weight average molecular weight of the block copolymer C (that is, the weight average molecular weight of the entire block copolymer C) is 100,000 to 300,000, the molecular weight distribution (Mw/Mn) of the block copolymer C (that is, the molecular weight distribution (Mw/Mn) of the entire block copolymer C) is 1.0 to 1.2, the content of the aromatic vinyl monomer unit of the block copolymer C is 12 to 22% by weight with respect to the total monomer units constituting the block copolymer C, and the cis/trans ratio of the conjugated diene monomer units constituting the conjugated diene polymer block ($D^c$) is 1.5 to 4 (i.e., cis/trans = 1.5 to 4).

The block copolymer D represented by the general formula (D), (Ar$^d$-D$^d$)$_n$-X, is preferably a block copolymer described below.

In the formula, Ar$^d$ represents an aromatic vinyl polymer block having a weight average molecular weight of 7,000 to 17,000, D$^d$ represents a conjugated diene polymer block having a weight average molecular weight of 60,000 to 190,000, n represents an integer of 2 or more, and X represents a coupling agent residue.

The weight average molecular weight of the block copolymer D (that is, the weight average molecular weight of the entire block copolymer D) is 100,000 to 400,000, the molecular weight distribution (Mw/Mn) of the block copolymer D is 1.0 to 1.2, the content of the aromatic vinyl monomer unit of the block copolymer D is 11 to 22% by weight with respect to the total monomer units constituting the block copolymer D, and the cis/trans ratio of the conjugated diene monomer units constituting the conjugated diene polymer block ($D^d$) is 1.5 to 4 (i.e., cis/trans = 1.5 to 4).

When the block copolymer D is a mixture of a plurality of polymers in which the values of n are different, it is sufficient that the molecular weight distribution (Mw/Mn) of the polymers of which the values of n can be determined to be the same falls within the above range. For example, when the block copolymer D is a mixture of a 2-arm branched polymer in which n = 2, a 3-arm branched polymer in which n = 3, and a 4-arm branched polymer in which n = 4, it is sufficient that the molecular weight distribution (Mw/Mn) of each of the 2-arm branched polymer, the 3-arm branched polymer, and the 4-arm branched polymer falls within the above range (i.e., it is sufficient that each of the branched polymers has a molecular weight distribution (Mw/Mn) of 1.0 to 1.2) .

In all of the block copolymer A, the block copolymer B, the block copolymer C, and the block copolymer D described above, not only the weight average molecular weights of the aromatic vinyl polymer block and the conjugated diene polymer block, and the molecular weight of the entire block copolymers fall within the specific ranges as defined above, but also the molecular weight distributions (Mw/Mn) of the entire block copolymers fall within a range of 1.0 to 1.2, and the cis/trans ratio of the conjugated diene monomer units constituting the conjugated diene polymer block falls within a range of 1.5 to 4.

Such an aromatic vinyl block copolymer composition comprising the block copolymer A, the block copolymer B, and at least one of the block copolymer C and the block copolymer D, in which the molecular weight distribution (Mw/Mn) and the cis/trans ratio fall within the specific ranges, suitably satisfies all of the (1) to (4) above, and thus can provide a film-shaped elastic body exhibiting a high stress and having a high stretchability and a large width.

(Block copolymer A)

The block copolymer A is a copolymer represented by the following general formula (A):

General formula (A) :        Ar1ª-Dª-Ar2ª

The aromatic vinyl polymer block (Ar1ª) and the aromatic vinyl polymer block (Ar2ª) are each a polymer block composed of an aromatic vinyl monomer unit obtained by polymerizing an aromatic vinyl monomer, as the main repeating unit.

The aromatic vinyl monomer used to constitute the aromatic vinyl monomer unit for the aromatic vinyl polymer block (Ar1ª) and the aromatic vinyl polymer block (Ar2ª) may be any aromatic vinyl compound. Examples of usable aromatic vinyl monomers include styrene, α-methylstyrene, 2-methylstyrene, 3-methylstyrene, 4-methylstyrene, 2-ethylstyrene, 3-ethylstyrene, 4-ethylstyrene, 2,4-diisopropylstyrene, 2,4-dimethylstyrene, 4-t-butylstyrene, 5-t-butyl-2-methylstyrene, 2-chlorostyrene, 3-chlorostyrene, 4-chlorostyrene, 4-bromostyrene, 2-methyl-4,6-dichlorostyrene, 2,4-dibromostyrene, vinylnaphthalene, and the like. Among these, use of styrene is preferred. These aromatic vinyl monomers can be used alone or in combination. Two of these aromatic vinyl polymer blocks, if present, may be composed of the same aromatic vinyl monomer unit, or may be composed of different aromatic vinyl monomer units.

The aromatic vinyl polymer block (Ar1ª) and the aromatic vinyl polymer block (Ar2ª) may contain another monomer unit as long as the aromatic vinyl monomer unit is the main repeating unit. Examples of monomers constituting the monomer unit other than the aromatic vinyl monomer unit which may be contained in the aromatic vinyl polymer block (Ar1ª) and the aromatic vinyl polymer block (Ar2ª) include conjugated diene monomers such as 1,3-butadiene and isoprene (2-methyl-1,3-butadiene), α,β-unsaturated nitrile monomers, unsaturated carboxylic acid or acid anhydride monomers, unsaturated carboxylic acid ester monomers, non-conjugated diene monomers, and the like. The content of the monomer unit other than the aromatic vinyl monomer unit in the aromatic vinyl polymer block (Ar1ª) and the aromatic vinyl polymer block (Ar2ª) is preferably 20% by weight or less, more preferably 10% by weight or less, particularly preferably substantially 0% by weight. In other words, the aromatic vinyl polymer block (Ar1ª) and the aromatic vinyl polymer block (Ar2ª) are preferably composed of substantially only one or two or more types of aromatic vinyl monomer units, and particularly preferably composed of only a styrene unit.

In the block copolymer A, the aromatic vinyl polymer block (Ar1ª) and the aromatic vinyl polymer block (Ar2ª) differ in weight average molecular weight.

The weight average molecular weight of the aromatic vinyl polymer block (Ar1ª) is 7,000 to 17,000, preferably 9,000 to 15,000, more preferably 10,000 to 14,000.

The weight average molecular weight of the aromatic vinyl polymer block (Ar2ª) is more than 20,000 and 300,000 or less, preferably 25,000 to 240,000, more preferably 30,000 to 190,000, still more preferably 35,000 to 180,000, and most preferably 35,000 to 99,000.

The conjugated diene polymer block (Dª) is a polymer block composed of a conjugated diene monomer unit obtained by polymerizing a conjugated diene monomer, as the main repeating unit.

The conjugated diene monomer used to constitute the conjugated diene monomer unit for the conjugated diene block (Dª) may be any conjugated diene compound. Examples thereof include 1,3-butadiene, isoprene, 2,3-dimethyl-1,3-butadiene, 2-chloro-1,3-butadiene, 1,3-pentadiene, 1,3-hexadiene, myrcene, farnesene, and the like. Among these, preferred is use of 1,3-butadiene and/or isoprene, and particularly preferred is use of isoprene. These conjugated diene monomers can be used alone or in combination. Furthermore, unsaturated bonds of the conjugated diene block (Dª) may be partially subjected to a hydrogenation reaction.

The conjugated diene block (Dª) may contain another monomer unit as long as the conjugated diene monomer unit is the main repeating unit. Examples of monomers constituting the monomer unit other than the conjugated diene monomer unit which may be contained in the conjugated diene block (Dª) include aromatic vinyl monomers such as styrene and α-methylstyrene, α,β-unsaturated nitrile monomers, unsaturated carboxylic acid monomers or acid anhydride monomers, unsaturated carboxylic acid ester monomers, non-conjugated diene monomers, and the like. The content of the monomer unit other than the conjugated diene monomer unit in the conjugated diene block (Dª) is preferably 20% by weight or less, more preferably 10% by weight or less, particularly preferably substantially 0% by weight. In other words, the conjugated diene block (Dª) is preferably composed of substantially only one or two or more types of conjugated diene monomer units, particularly preferably composed of only isoprene units.

The cis/trans ratio of the conjugated diene monomer units constituting the conjugated diene polymer block (Dª) is 1.5 to 4 (i.e., cis/trans = 1.5 to 4), preferably 1.8 to 3, more preferably 2 to 2.7.

The vinyl bond content of the conjugated diene monomer unit constituting the conjugated diene polymer block (Dª) is not particularly limited, but is preferably 1 to 20 mol%, more preferably 2 to 15 mol%, particularly preferably 3 to 10 mol%.

The weight average molecular weight of the conjugated diene polymer block (Dª) is 50,000 to 200,000, preferably 70,000 to 150,000, more preferably from 80,000 to 120,000.

The weight average molecular weight of the block copolymer A (that is, the weight average molecular weight of the entire block copolymer A) is 80,000 to 400,000, preferably 110,000 to 330,000, more preferably 130,000 to 270,000.

The molecular weight distribution (Mw/Mn) of the block copolymer A (that is, the molecular weight distribution (Mw/Mn) of the entire block copolymer A) is 1.0 to 1.2, preferably 1.005 to 1.1, more preferably 1.01 to 1.05.

In the present invention, the weight average molecular weight of each polymer block, and the weight average molecular weight, the number average molecular weight, and the molecular weight distribution (Mw/Mn) of the entire block polymer are determined as values in terms of polystyrene by measurement of high performance liquid chromatography.

Further, the weight average molecular weight of each polymer block and the weight average molecular weight of the entire block polymer can be controlled by adjusting the amount of monomers to be used for forming its corresponding polymer block, the amount of a polymerization initiator, and the amount of a polymerization terminator to be used to obtain the block copolymer by the polymerization reaction.

The content of the aromatic vinyl monomer unit of the block copolymer A is 39 to 85% by weight, preferably 40 to 76% by weight, more preferably 41 to 70% by weight with respect to the total monomer units constituting the block copolymer A.

It is preferable that the block copolymer A be obtained by a production method in which a polymer block is formed by sequentially polymerizing an aromatic vinyl monomer, a conjugated diene monomer, and then an aromatic vinyl monomer in this order. Therefore, it is preferable that the block copolymer A do not contain a residue of a coupling agent. Details of such a production method will be described later. However, the production method is not particularly limited as long as the copolymer having a structure represented by $Ar1^a$-$D^a$-$Ar2^a$ can be produced. The copolymer may be produced using a coupling agent, and may contain a coupling agent residue.

(Block copolymer B)

The block copolymer B is a copolymer represented by the following general formula (B):

General formula (B):     $Ar1^b$-$D^b$-$Ar2^b$

The aromatic vinyl polymer block ($Ar1^b$) and the aromatic vinyl polymer block ($Ar2^b$) are each a polymer block composed of an aromatic vinyl monomer unit obtained by polymerizing an aromatic vinyl monomer, as the main repeating unit.

The aromatic vinyl monomer used for constituting the aromatic vinyl monomer unit in the aromatic vinyl polymer block ($Ar1^b$) and the aromatic vinyl polymer block ($Ar2^b$) is not particularly limited as long as it is an aromatic vinyl compound, and examples thereof include the same aromatic vinyl monomers as those in the aromatic vinyl polymer block ($Ar1^a$) and the aromatic vinyl polymer block ($Ar2^a$) described above.

The aromatic vinyl polymer block ($Ar1^b$) and the aromatic vinyl polymer block ($Ar2^b$) may include another monomer unit, as the monomer constituting the monomer unit other than the aromatic vinyl monomer unit as long as the aromatic vinyl monomer unit is the main repeating unit, and examples of monomers constituting the monomer unit other than the aromatic vinyl monomer unit include the same monomers as those in the aromatic vinyl polymer block ($Ar1^a$) and the aromatic vinyl polymer block ($Ar2^a$) described above, and the content thereof may be the same as in the aromatic vinyl polymer block described above. Further, it is preferable that each of the aromatic vinyl polymer block ($Ar1^b$) and the aromatic vinyl polymer block ($Ar2^b$) be also substantially composed of only one or two or more types of aromatic vinyl monomer units, and it is particularly preferable that these be composed of only a styrene unit.

In the block copolymer B, the aromatic vinyl polymer block ($Ar1^b$) and the aromatic vinyl polymer block ($Ar2^b$) both have a weight average molecular weight ranging from 7,000 to 17,000. The weight average molecular weights of the aromatic vinyl polymer block ($Ar1^b$) and the aromatic vinyl polymer block ($Ar2^b$) are 7,000 to 17,000, preferably 9,000 to 15,000, more preferably 10,000 to 14,000.

The weight average molecular weights of the aromatic vinyl polymer block ($Ar1^b$) and the aromatic vinyl polymer block ($Ar2^b$) may be equal to or different from each other as long as they fall within the above ranges, but the ratio of "the weight average molecular weight of the aromatic vinyl polymer block ($Ar1^b$)/the weight average molecular weight of the aromatic vinyl polymer block ($Ar2^b$)" is preferably 0.7 to 1.3, more preferably 0.8 to 1.2, more preferably 0.9 to 1.1, still more preferably 0.95 to 1.05, and the weight average molecular weights are most preferably substantially equal to each other.

For the weight average molecular weights of the aromatic vinyl polymer block ($Ar1^b$) and the aromatic vinyl polymer block ($Ar2^b$) relative to the weight average molecular weight of the aromatic vinyl polymer block ($Ar1^a$) which has a relatively small weight average molecular weight in the block copolymer A, the ratio of "the weight average molecular weight of the aromatic vinyl polymer block ($Ar1^b$)/the weight average molecular weight of the aromatic vinyl polymer block ($Ar1^a$)" or "the weight average molecular weight of the aromatic vinyl polymer block ($Ar2^b$)/the weight average molecular weight of the aromatic vinyl polymer block ($Ar1^a$)" is preferably 0.7 to 1.3, more preferably 0.8 to 1.2, more preferably 0.9 to 1.1, still more preferably 0.95 to 1.05, and the weight average molecular weights of these polymer blocks are most preferably substantially equal to each other.

The conjugated diene polymer block ($D^b$) is a polymer block composed of a conjugated diene monomer unit obtained by polymerizing a conjugated diene monomer, as the main repeating unit.

The conjugated diene monomer used for constituting the conjugated diene monomer unit of the conjugated diene polymer block ($D^b$) is not particularly limited as long as it is a conjugated diene compound, and examples thereof include the same conjugated diene monomers as those listed in the conjugated diene polymer block ($D^a$) described above.

The conjugated diene polymer block ($D^b$) may include another monomer unit as long as the conjugated diene monomer unit is the main repeating unit, and examples of monomers constituting the monomer unit other than the conjugated diene monomer unit include the same monomers as those listed in the conjugated diene polymer block ($D^a$) described above, and the content thereof and vinyl bond content may be the same as in the conjugated diene polymer block described above. Further, it is preferable that the conjugated diene polymer block ($D^b$) be also substantially composed of only one or two or more types of conjugated diene monomer units, and it is particularly preferable that it be composed of only isoprene units.

The cis/trans ratio of the conjugated diene monomer units constituting the conjugated diene polymer block ($D^b$) is 1.5 to 4 (i.e., cis/trans = 1.5 to 4), preferably 1.8 to 3, more preferably 2 to 2.7.

It is preferable that the vinyl bond content of the conjugated diene polymer block ($D^b$) be substantially equal to the vinyl bond content of the conjugated diene polymer block ($D^a$) of the block copolymer A.

The weight average molecular weight of the conjugated diene polymer block ($D^b$) is 50,000 to 200,000, preferably 70,000 to 150,000, more preferably from 80,000 to 120,000.

For the weight average molecular weight of the conjugated diene polymer block ($D^b$) relative to the weight average molecular weight of the conjugated diene polymer block ($D^a$) in the block copolymer A, the ratio of "the weight average molecular weight of the conjugated diene polymer block ($D^b$)/the weight average molecular weight of the conjugated diene polymer block ($D^a$)" is not particularly limited, but is preferably 0.5 to 2.0, more preferably 0.7 to 1.3, and the weight average molecular weights of these polymer blocks are still more preferably substantially equal to each other.

The weight average molecular weight of the block copolymer B (that is, the weight average molecular weight of the entire block copolymer B) is preferably 75,000 to 300,000, more preferably 80,000 to 180,000, still more preferably from 90,000 to 150,000.

The molecular weight distribution (Mw/Mn) of the block copolymer B (that is, the molecular weight distribution (Mw/Mn) of the entire block copolymer B) is 1.0 to 1.2, preferably 1.005 to 1.1, more preferably 1.01 to 1.05.

The content of the aromatic vinyl monomer unit of the block copolymer B is 21 to 30% by weight, preferably 22 to 29% by weight, more preferably 23 to 28% by weight with respect to the total monomer units constituting the block copolymer B.

It is preferable that the block copolymer B be obtained by a production method in which a polymer block is formed by sequentially polymerizing an aromatic vinyl monomer, a conjugated diene monomer, and then an aromatic vinyl monomer in this order. Therefore, it is preferable that the block copolymer B do not contain a residue of a coupling agent. Details of such a production method will be described later. However, the production method is not particularly limited as long as the copolymer having a structure represented by $Ar1^b$-$D^b$-$Ar2^b$ can be produced. The copolymer may be produced using a coupling agent, and may contain a coupling agent residue.

(Block copolymer C)

The block copolymer C is a copolymer represented by the following general formula (C):

General formula (C) :　　　　$Ar1^c$-$D^c$-$Ar2^c$

The aromatic vinyl polymer block ($Ar1^c$) and the aromatic vinyl polymer block ($Ar2^c$) are each a polymer block composed of an aromatic vinyl monomer unit obtained by polymerizing an aromatic vinyl monomer, as the main repeating unit.

The aromatic vinyl monomer used to constitute the aromatic vinyl monomer unit in the aromatic vinyl polymer block ($Ar1^c$) and the aromatic vinyl polymer block ($Ar2^c$) is not particularly limited as long as it is an aromatic vinyl compound, and examples thereof include the same aromatic vinyl monomers as those listed in the aromatic vinyl polymer block ($Ar1^a$) and the aromatic vinyl polymer block ($Ar2^a$) described above.

The aromatic vinyl polymer block ($Ar1^c$) and the aromatic vinyl polymer block ($Ar2^c$) may include another monomer unit as long as the aromatic vinyl monomer unit is the main repeating unit, and examples of monomers constituting the monomer unit other than the aromatic vinyl monomer unit include the same monomers as those listed in the aromatic vinyl polymer block ($Ar1^a$) and the aromatic vinyl polymer block ($Ar2^a$) described above, and the content thereof may be the same as in the aromatic vinyl polymer block described above. Further, it is preferable that each of the aromatic vinyl polymer block ($Ar1^c$) and the aromatic vinyl polymer block ($Ar2^c$) be also substantially composed of only one or two or more types of aromatic vinyl monomer units, and it is particularly preferable that these be composed of only a styrene unit.

In the block copolymer C, the aromatic vinyl polymer block ($Ar1^c$) and the aromatic vinyl polymer block ($Ar2^c$) both have a weight average molecular weight ranging from 7,000 to 17,000. The weight average molecular weights of the aromatic vinyl polymer block (Ar1$^c$) and the aromatic vinyl polymer block (Ar2$^c$) are 7,000 to 17,000, preferably 8,000 to 15,000, more preferably 9,000 to 14,000.

The weight average molecular weights of the aromatic vinyl polymer block (Ar1$^c$) and the aromatic vinyl polymer block (Ar2$^c$) may be equal to or different from each other as long as they fall within the above ranges, but the ratio of "the weight average molecular weight of the aromatic vinyl polymer block (Ar1$^c$)/the weight average molecular weight of the aromatic vinyl polymer block (Ar2$^c$)" is preferably 0.7 to 1.3, more preferably 0.8 to 1.2, more preferably 0.9 to 1.1, still more preferably 0.95 to 1.05, and the weight average molecular weights are most preferably substantially equal to each other.

The conjugated diene polymer block (D$^c$) is a polymer block composed of a conjugated diene monomer unit obtained by polymerizing a conjugated diene monomer, as the main repeating unit.

The conjugated diene monomer used for constituting the conjugated diene monomer unit of the conjugated diene polymer block (D$^c$) is not particularly limited as long as it is a conjugated diene compound, and examples thereof include the same conjugated diene monomers as those listed in the conjugated diene polymer block (D$^a$) described above.

Further, the conjugated diene polymer block (D$^c$) may include another monomer unit as long as the conjugated diene monomer unit is the main repeating unit, and examples of monomers constituting the monomer unit other than the conjugated diene monomer unit include the same monomers as those listed in the conjugated diene polymer block (D$^a$) described above, and the content thereof and the vinyl bond content may be the same as in the conjugated diene polymer block described above. Further, it is preferable that the conjugated diene polymer block (D$^c$) be also substantially composed of only one or two or more types of conjugated diene monomer units, and it is particularly preferable that it be composed of only isoprene units.

The cis/trans ratio of the conjugated diene monomer units constituting the conjugated diene polymer block (D$^c$) is 1.5 to 4 (i.e., cis/trans = 1.5 to 4), preferably 1.8 to 3, more preferably 2.2 to 2.6.

The vinyl bond content of the conjugated diene monomer unit constituting the conjugated diene polymer block (D$^c$) is not particularly limited, but is preferably 1 to 20 mol%, more preferably 2 to 15 mol%, particularly preferably 3 to 10 mol%.

The weight average molecular weight of the conjugated diene polymer block (D$^c$) is 80,000 to 250,000, preferably 120,000 to 200,000, more preferably 150,000 to 185,000.

The weight average molecular weight of the block copolymer C (that is, the weight average molecular weight of the entire block copolymer C) is preferably 100,000 to 300,000, more preferably 130,000 to 240,000, still more preferably 160,000 to 200,000.

The molecular weight distribution (Mw/Mn) of the block copolymer C (that is, the molecular weight distribution (Mw/Mn) of the entire block copolymer C) is 1.0 to 1.2, preferably 1.005 to 1.1, more preferably 1.01 to 1.05.

The content of the aromatic vinyl monomer unit of the block copolymer C is 12 to 22% by weight, preferably 13 to 21% by weight, more preferably 15 to 20% by weight with respect to the total monomer units constituting the block copolymer C.

It is preferable that the block copolymer C be obtained by a production method in which a polymer block is formed by sequentially polymerizing an aromatic vinyl monomer, a conjugated diene monomer, and then an aromatic vinyl monomer in this order. Therefore, it is preferable that the block copolymer C do not contain a residue of a coupling agent. Details of such a production method will be described later.

(Block copolymer D)

The block copolymer D is a copolymer represented by the following general formula (D):

General formula (D) :     $(Ar^d\text{-}D^d)_n\text{-}X$

The block copolymer D has a structure in which "n" of diblock structures (Ar$^d$-D$^d$) are bonded to each other through a residue (X) of a coupling agent. "n" in the general formula (D) represents the number of branches in the block copolymer D. The block copolymer D may be a mixture of two or more block copolymers composed of different numbers of diblock structures bonded to each other. "n" is an integer of two or more, preferably an integer of 2 to 8, more preferably an integer of 2 to 4, still more preferably 2. The value of n is the average of the values of "n" in a plurality of the block copolymers D. The residue (X) of the coupling agent is not particularly limited as long as it is a residue of an n-valent coupling agent (coupling agent whose valent number is "n"), but is preferably a residue of a silicon atom-containing coupling agent, and more preferably a residue of a halogenated silane or an alkoxysilane. Examples of the coupling agent constituting the residue of the coupling agent include those described later.

As the block copolymer D, among them, it is particularly preferred to be a mixture of a block copolymer D1 having "n" of 2, a block copolymer D2 having "n" of 3, and a block copolymer D3 having "n" of 4. The weight ratio (D1/D2/D3) of the block copolymers D1 to D3 in this case is preferably 40 to 80/30 to 10/30 to 10.

The aromatic vinyl polymer block (Ar$^d$) is a polymer block composed of an aromatic vinyl monomer unit obtained by polymerizing an aromatic vinyl monomer, as the main repeating unit.

The aromatic vinyl monomer used to constitute the aromatic vinyl monomer unit in the aromatic vinyl polymer block ($Ar^d$) is not particularly limited as long as it is an aromatic vinyl compound, and examples thereof include the same aromatic vinyl monomers as those listed in the aromatic vinyl polymer block ($Ar1^a$) and the aromatic vinyl polymer block ($Ar2^a$) described above.

The aromatic vinyl polymer block ($Ar^d$) may include another monomer unit as long as the aromatic vinyl monomer unit is the main repeating unit, and examples of monomers constituting the monomer unit other than the aromatic vinyl monomer unit include the same monomers as those listed in the aromatic vinyl polymer block ($Ar1^a$) and the aromatic vinyl polymer block ($Ar2^a$) described above, and the content thereof may be the same as in the aromatic vinyl polymer block described above. Further, the aromatic vinyl polymer block in the block copolymer D may be composed of the same aromatic vinyl monomer unit, or may be composed of different aromatic vinyl monomer units. In addition, it is preferable that the aromatic vinyl polymer block ($Ar^d$) be also substantially composed of only one or two or more types of aromatic vinyl monomer units, and it is particularly preferable that it be composed of only a styrene unit.

The weight average molecular weight of the aromatic vinyl polymer block ($Ar^d$) is 7,000 to 17,000, preferably 8,000 to 15,000, more preferably 8,500 to 14,000.

The weight average molecular weights of a plurality of aromatic vinyl polymer blocks ($Ar^d$) in the block copolymer D may be equal to or different from each other as long as they fall within the above ranges, but are preferably substantially equal to each other.

The conjugated diene polymer block ($D^d$) is a polymer block composed of a conjugated diene monomer unit obtained by polymerizing a conjugated diene monomer, as the main repeating unit.

The conjugated diene monomer used for constituting the conjugated diene monomer unit of the conjugated diene polymer block ($D^d$) is not particularly limited as long as it is a conjugated diene compound, and examples thereof include the same conjugated diene monomers as those listed in the conjugated diene polymer block ($D^a$) described above. Further, the conjugated diene polymer block in the block copolymer D may be composed of the same conjugated diene monomer unit, or may be composed of different conjugated diene monomer units.

Further, the conjugated diene polymer block ($D^d$) may include another monomer unit as long as the conjugated diene monomer unit is the main repeating unit, and examples of monomers constituting the monomer unit other than the conjugated diene monomer unit include the same monomers as those listed in the conjugated diene polymer block ($D^a$) described above, and the content thereof and the vinyl bond content may be the same as in the conjugated diene polymer block described above. Further, it is preferable that the conjugated diene polymer block ($D^d$) be also substantially composed of only one or two or more types of conjugated diene monomer units, and it is particularly preferable that it be composed of only isoprene units.

The cis/trans ratio of the conjugated diene monomer units constituting the conjugated diene polymer block ($D^d$) is 1.5 to 4 (i.e., cis/trans = 1.5 to 4), preferably 1.8 to 3, more preferably 2 to 2.7.

The vinyl bond content of the conjugated diene monomer unit constituting the conjugated diene polymer block ($D^d$) is not particularly limited, but is preferably 1 to 20 mol%, more preferably 2 to 15 mol%, particularly preferably 3 to 10 mol%.

The weight average molecular weight of the conjugated diene polymer blocks ($D^d$) is 6,0000 to 190,000, preferably 70,000 to 160,000, more preferably 80,000 to 140,000.

The weight average molecular weights of the conjugated diene polymer blocks ($D^d$) in the block copolymer D may be equal or different from each other as long as they fall within the above range, but are preferably substantially equal to each other.

The weight average molecular weight of the block copolymer D (that is, the weight average molecular weight of the entire block copolymer D) is 100,000 to 400,000, preferably 140,000 to 350,000, more preferably 180,000 to 320,000.

The molecular weight distribution (Mw/Mn) of the block copolymer D is 1.0 to 1.2, preferably 1.005 to 1.1, more preferably 1.01 to 1.05. When the block copolymer D is a mixture of a plurality of polymers in which the values of n are different, it is sufficient that the molecular weight distribution (Mw/Mn) of the polymers of which the values of n can be determined to be the same falls within the above range. For example, when the block copolymer D is a mixture of a 2-arm branched polymer in which n = 2, a 3-arm branched polymer in which n = 3, and a 4-arm branched polymer in which n = 4, it is sufficient that the molecular weight distribution (Mw/Mn) of each of the 2-arm branched polymer, the 3-arm branched polymer, and the 4-arm branched polymer falls within the above range (i.e., it is sufficient that each of the branched polymers has a molecular weight distribution (Mw/Mn) of 1.0 to 1.2) .

The content of the aromatic vinyl monomer unit of the block copolymer D is 11 to 22% by weight, preferably 11.5 to 20% by weight, more preferably 12 to 18% by weight with respect to the total monomer units constituting the block copolymer D.

It is preferable that the block copolymer D be obtained by a production method in which the aromatic vinyl monomer and the conjugated diene monomer are polymerized to form diblock chains, and the diblock chains and a coupling agent are reacted to perform coupling. Thus, the block copolymer D contains residues of the coupling agent.

Details of such a production method will be described later.

(Block copolymer E)

The aromatic vinyl block copolymer composition used in the present invention may further contain a block copolymer E. The block copolymer E is a copolymer represented by the following general formula (E):

General formula (E):     $Ar^e$-$D^e$

(In the formula, $Ar^e$ represents an aromatic vinyl polymer block, and $D^e$ represents a conjugated diene polymer block.)

The aromatic vinyl polymer block ($Ar^e$) is a polymer block composed of an aromatic vinyl monomer unit obtained by polymerizing an aromatic vinyl monomer, as the main repeating unit.

The aromatic vinyl monomer used to constitute the aromatic vinyl monomer unit in the aromatic vinyl polymer block ($Ar^e$) is not particularly limited as long as it is an aromatic vinyl compound, and examples thereof include the same aromatic vinyl monomers as those listed in the aromatic vinyl polymer block ($Ar1^a$) and the aromatic vinyl polymer block ($Ar2^a$) described above.

The aromatic vinyl polymer block ($Ar^e$) may include another monomer unit as long as the aromatic vinyl monomer unit is the main repeating unit, and examples of monomers constituting the monomer unit other than the aromatic vinyl monomer unit include the same monomers as those listed in the aromatic vinyl polymer block ($Ar1^a$) and the aromatic vinyl polymer block ($Ar2^a$) described above, and the content thereof may be the same as in the aromatic vinyl polymer block described above. In addition, it is preferable that the aromatic vinyl polymer block ($Ar^e$) be also substantially composed of only one or two or more types of aromatic vinyl monomer units, and it is particularly preferable that it be composed of only a styrene unit.

The weight average molecular weight of the aromatic vinyl polymer block ($Ar^e$) is 7,000 to 17,000, preferably 8,000 to 15,000, more preferably 8,500 to 14,000.

For the weight average molecular weight of the aromatic vinyl polymer block ($Ar^e$) relative to the weight average molecular weight of the aromatic vinyl polymer block ($Ar^d$) in the block copolymer D, the ratio of "the weight average molecular weight of the aromatic vinyl polymer block ($Ar^e$)/the weight average molecular weight of the aromatic vinyl polymer block ($Ar^d$)" is preferably 0.7 to 1.3, more preferably 0.8 to 1.2, more preferably 0.9 to 1.1, still more preferably 0.95 to 1.05, and the weight average molecular weights of these polymer blocks are most preferably substantially equal to each other.

The conjugated diene polymer block ($D^e$) is a polymer block composed of a conjugated diene monomer unit obtained by polymerizing a conjugated diene monomer, as the main repeating unit.

The conjugated diene monomer used for constituting the conjugated diene monomer unit of the conjugated diene polymer block ($D^e$) is not particularly limited as long as it is a conjugated diene compound, and examples thereof include the same conjugated diene monomers as those listed in the conjugated diene polymer block ($D^a$) described above.

The conjugated diene polymer block ($D^e$) may include another monomer unit as long as the conjugated diene monomer unit is the main repeating unit, and examples of monomers constituting the monomer unit other than the conjugated diene monomer unit include the same monomers as those listed in the conjugated diene polymer block ($D^a$) described above, and the content thereof and the vinyl bond content may be the same as in the conjugated diene polymer block described above. Further, it is preferable that the conjugated diene polymer block ($D^e$) be also substantially composed of only one or two or more types of conjugated diene monomer units, and it is particularly preferable that it be composed of only isoprene units.

The cis/trans ratio of the conjugated diene monomer units constituting the conjugated diene polymer block ($D^e$) is 1.5 to 4 (i.e., cis/trans = 1.5 to 4), preferably 1.8 to 3, more preferably 2 to 2.7.

It is preferable that the vinyl bond content of the conjugated diene polymer block ($D^e$) be substantially equal to the vinyl bond content of the conjugated diene polymer block ($D^d$) of the block copolymer D.

The weight average molecular weight of the conjugated diene polymer block ($D^e$) is 60,000 to 190,000, preferably 70,000 to 160,000, more preferably from 80,000 to 140,000.

For the weight average molecular weight of the conjugated diene polymer block ($D^e$) relative to the weight average molecular weight of the conjugated diene polymer block ($D^d$) of the block copolymer D, the ratio of "the weight average molecular weight of the conjugated diene polymer block ($D^e$)/the weight average molecular weight of the conjugated diene polymer block ($D^d$)" is not particularly limited, but is preferably 0.7 to 1.3, more preferably 0.8 to 1.2, more preferably 0.9 to 1.1, still more preferably 0.95 to 1.05, and the weight average molecular weights of these polymer blocks are most preferably substantially equal to each other.

The weight average molecular weight of the block copolymer E (that is, the weight average molecular weight of the entire block copolymer E) is 50,000 to 200,000, preferably 70,000 to 170,000, more preferably 80,000 to 150,000.

The molecular weight distribution (Mw/Mn) of the block copolymer E (that is, the molecular weight distribution (Mw/Mn) of the entire block copolymer E) is 1.0 to 1.2, preferably 1.005 to 1.1, more preferably 1.01 to 1.05.

The content of the aromatic vinyl monomer unit of the block copolymer E is 11 to 22% by weight, preferably 11.5 to 20% by weight, more preferably 12 to 18% by weight with respect to the total monomer units constituting the block copolymer E.

The aromatic vinyl block copolymer composition used in the present invention comprises the block copolymer A, the block copolymer B, and at least one selected from the group consisting of the block copolymer C and the block copolymer D, and optionally further comprises the block copolymer E. The weight ratio (A/B/C+D) of the block copolymers A to D in the aromatic vinyl block copolymer composition used in the present invention is preferably 5 to 50/10 to 55/75 to 25, more preferably 10 to 30/25 to 50/60 to 30, still more preferably 15 to 25/33 to 49/45 to 26.

The total content of the block copolymer A, the block copolymer B, and at least one selected from the group consisting of the block copolymer C and the block copolymer D in the aromatic vinyl block copolymer composition used in the present invention is preferably 70 to 100% by weight, more preferably 80 to 99.7% by weight, still more preferably 84 to 99.5% by weight, most preferably 88 to 99% by weight.

When the aromatic vinyl block copolymer composition used in the present invention further comprises the block copolymer E, the content of the block copolymer E in the aromatic vinyl block copolymer composition including the block copolymers A to D is preferably 1 to 15% by weight, more preferably 2 to 10% by weight, still more preferably 3 to 9% by weight.

(Method for producing aromatic vinyl block copolymer composition)

The block copolymers A to E, which constitute the aromatic vinyl block copolymer composition used in the present invention, can be produced according to a conventional method, and examples of the most general production method include a method in which an aromatic vinyl monomer and a conjugated diene monomer are sequentially polymerized by an anion living polymerization method to form polymer blocks, and if necessary, a coupling agent is reacted to perform coupling. Alternatively, a method of forming polymer blocks by sequentially polymerizing an aromatic vinyl monomer, a conjugated diene monomer, and then an aromatic vinyl monomer in this order may be used. Alternatively, polymer blocks can be produced by separately producing each polymer according to a conventional polymerization method, if necessary, blending other polymer components and the like, and then mixing them according to a conventional method such as kneading or solution mixing. Further, as will be described later, it is also possible to simultaneously produce block copolymers A and B or to simultaneously produce block copolymers D and E.

The block copolymer A and the block copolymer B are preferably produced by a method in which polymer blocks are formed by sequentially polymerizing an aromatic vinyl monomer, a conjugated diene monomer, and then an aromatic vinyl monomer in this order.

More particularly, the block copolymer A and the block copolymer B are preferably produced by the following production method. In other words, preferred is a production method comprising:

a first polymerization step of obtaining a solution containing aromatic vinyl polymer block chains by polymerizing monomers containing an aromatic vinyl monomer in a solvent in the presence of a polymerization initiator,

a second polymerization step of obtaining a solution containing diblock chains by adding monomers containing a conjugated diene monomer to the solution containing aromatic vinyl polymer block chains, and performing polymerization,

a third polymerization step of obtaining a solution containing triblock chains by adding monomers containing an aromatic vinyl monomer to the solution containing diblock chains, and performing polymerization,

a termination step by adding, to the solution containing triblock chains, a polymerization terminator in an amount which becomes less than 1 molar equivalents with respect to the active terminals of the triblock chains, and deactivating part of the active terminals to obtain a solution containing the block copolymer B and triblock chains, and

a fourth polymerization step of obtaining a solution containing the block copolymer A and the block copolymer B by adding monomers containing an aromatic vinyl monomer to the solution containing triblock chains and the block copolymer B and performing polymerization.

However, the method for producing the block copolymer A and the block copolymer B is not limited to those described above, and any method can be used as long as the block copolymer A and the block copolymer B can be obtained. For example, these polymers may be produced by a method in which an aromatic vinyl monomer and a conjugated diene monomer are polymerized to form diblock structures, the resulting diblock structures are subjected to a coupling reaction using a coupling agent. In this case, the block copolymer A and the block copolymer B typically contain a residue of the coupling agent.

In the production method above, first, the monomers containig an aromatic vinyl monomer as the main component are polymerized in a solvent in the presence of a polymerization initiator (first polymerization step). From the viewpoint of the capability of properly controlling the molecular weight distribution (Mw/Mn) of the obtained block copolymer to fall within the above ranges, in the aromatic vinyl monomer used in the polymerization, the amount of deactivation components, such as water, which deactivate the polymerization initiator is preferably controlled to be 1 × $10^{-3}$ mol/kg or less. In the following aromatic vinyl monomer used in the production of each block copolymer, from the viewpoint of properly controlling the molecular weight distribution (Mw/Mn) of each obtained block copolymer to fall within the above range, the amount of deactivation components is preferably controlled to be within the above range.

The polymerization initiator to be used can be organic alkali metal compounds, organic alkaline earth metal compounds, organic lanthanoid rare earth metal compounds, and the like, which are known to usually have anionic polymerization activity to aromatic vinyl monomers and conjugated diene monomers. For the organic alkali metal compounds, particularly suitably used are organic lithium compounds having one or more lithium atoms in the molecule. Specific examples thereof include organic monolithium compounds such as ethyllithium, n-propyllithium, isopropyllithium, n-butyllithium, sec-butyllithium, t-butyllithium, hexyllithium, phenyllithium, stilbenelithium, dialkylaminolithium, diphenylaminolithium, and ditrimethylsilylaminolithium; organic dilithium compounds such as methylenedilithium, tetramethylenedilithium, hexamethylenedilithium, isoprenyldilithium, and 1,4-dilithio-ethylcyclohexane; organic trilithium compounds such as 1,3,5-trilithiobenzene; and the like. Among these, organic monolithium compounds are particularly suitably used.

Examples of the organic alkaline earth metal compounds used as the polymerization initiator include n-butylmagnesium bromide, n-hexylmagnesium bromide, ethoxycalcium, calcium stearate, t-butoxystrontium, ethoxybarium, isopropoxybarium, ethylmercaptobarium, t-butoxybarium, phenoxybarium, diethylaminobarium, barium stearate, ethylbarium, and the like. Specific examples of other polymerization initiators include those which form a homogeneous system in an organic solvent to have living polymerizability, such as composite catalysts of lanthanoid rare earth metal compounds (including neodymium, samarium, gadolinium, and the like)/alkylaluminum/alkylaluminum halide/alkylaluminum hydride; metallocene catalysts containing titanium, vanadium, samarium, gadolinium, or the like. These polymerization initiators may be used alone or in combination as a mixture.

The amount of the polymerization initiator to be used may be determined according to the target molecular weight, and is not particularly limited. The amount is preferably 0.01 to 20 mmol, more preferably 0.05 to 15 mmol, still more preferably 0.1 to 10 mmol with respect to 100 g of the total monomers.

The solvent used in polymerization can be any solvent inactive to the polymerization initiator. For example, a linear hydrocarbon solvent, a cyclic hydrocarbon solvent, or a mixed solvent thereof is used. Examples of the linear hydrocarbon solvent include linear alkanes and alkenes having 4 to 6 carbon atoms such as n-butane, isobutane, 1-butene, isobutylene, trans-2-butene, cis-2-butene, 1-pentene, trans-2-pentene, cis-2-pentene, n-pentane, isopentane, neopentane, and n-hexane. Specific examples of the cyclic hydrocarbon solvent include aromatic compounds such as benzene, toluene, and xylene; and alicyclic hydrocarbon compounds such as cyclopentane and cyclohexane. These solvents may be used alone or in combination as a mixture.

Although not particularly limited, the amount of the solvent used in polymerization is preferably set such that the content of the block copolymer in the finally obtained block copolymer solution falls within the range of preferably 5 to 60% by weight, preferably 15 to 55% by weight, particularly preferably 25 to 50% by weight.

A Lewis base compound may be added to a reactor used in polymerization to control the structures of the polymer blocks. Examples of the Lewis base compound include ethers such as tetrahydrofuran, diethyl ether, dioxane, ethylene glycol dimethyl ether, ethylene glycol dibutyl ether, diethylene glycol dimethyl ether, and diethylene glycol dibutyl ether; tertiary amines such as tetramethylethylenediamine, trimethylamine, triethylamine, pyridine, and quinuclidine; alkali metal alkoxides such as potassium t-amyloxide and potassium t-butyloxide; phosphines such as triphenylphosphine; and the like. These Lewis base compounds may be used alone or in combination, and are appropriately selected in the range not impairing the object of the present invention.

The Lewis base compound can be added to the polymerization reaction at any timing, which may be appropriately determined according to the structures of the block copolymers. For example, the Lewis base compounds may be preliminarily added before polymerization is started, or may be added after the polymer blocks are partially polymerized. Furthermore, the Lewis base compound may be preliminarily added before polymerization is started, and may be further added after the polymer blocks are partially polymerized. From the viewpoint of properly controlling the molecular weight distribution (Mw/Mn) of the obtained block copolymer and the cis/trans ratio of the conjugated diene block to fall within the above ranges, the amount of the Lewis base compound to be used may be determined according to the type of the Lewis base compound, the type of the solvent, and the like to be used. For example, when a linear hydrocarbon solvent, a cyclic hydrocarbon solvent, or a mixed solvent thereof is used and tetramethylethylenediamine is used as the Lewis base compound, the amount of the Lewis compound is preferably 0.020 to 0.200 mol, more preferably 0.025 to 0.110 mol, still more preferably 0.028 to 0.06 mol per mol of the polymerization initiator.

The temperature for the polymerization reaction is preferably 30 to 90°C, more preferably 35 to 85°C, still more preferably 40 to 80°C. Although the time needed for polymerization varies according to the condition, the time is preferably preferably 0.5 to 10 hours, more preferably 1 to 8 hours, still more preferably 2 to 5 hours. Although the polymerization may be carried out at a pressure enough to maintain the monomer and the solvent in liquid phases at a polymerization temperature in the above range, the polymerization pressure is preferably 1 kPa or more, more preferably 10 kPa or more, still more preferably 50 kPa or more.

When the polymerization reaction is carried out, the polymerization is preferably carried out while the liquid phase constituting the polymerization reaction system is being stirred. In this case, the polymerization is preferably carried out under a condition that allows the Reynolds number for stirring of the liquid phase constituting the polymerization reaction system to be 100 or more, more preferably carried out under a condition that allows the Reynolds number for stirring to be 500 to 1,000,000, still more preferably carried out under a condition that allows the Reynolds number for stirring to be 1,000 to 100,000. Alternatively, it is preferred that the liquid phase constituting the polymerization reaction system be stirred using a stirring blade, and preferably the stirring is carried out with a power used for rotating the stirring blade of 0.5 kW/m$^3$ or more, more preferably the stirring is carried out with a power used for rotating the stirring blade of 1 to 20 kW/m$^3$, and still more preferably the stirring is carried out with a power used for rotating the stirring blade of 2 to 10 kW/m$^3$. When the polymerization is carried out under a condition that allows the Reynolds number for stirring or the power used for rotating a stirring blade to fall within the above ranges, the molecular weight distribution (Mw/Mn) of the obtained block copolymer can be properly controlled to fall with the above ranges. The Reynolds number (Re) for stirring is a value represented by Re = $\rho nd^2/\mu$, where $\rho$ refers to the density (kg/m$^3$) of the mixed solution to be stirred, d refers to the diameter (m) of the stirring blade, $\mu$ refers to the viscosity (kg/(m·s)) of the liquid phase to be stirred, and n refers to the rotation speed (l/s = N/60) of the stirring blade. In the polymerization of the block copolymers described below, from the viewpoint of properly controlling the molecular weight distribution (Mw/Mn) of each obtained block copolymer to fall within the above ranges, it is preferred that the polymerization be carried out under a condition that allows the Reynolds number for stirring or the power used for rotating a stirring blade to fall within the above ranges.

A solution containing aromatic vinyl polymer block chains can be prepared by polymerizing monomers containing an aromatic vinyl monomer as the main component in the presence of the polymerization initiator under the condition described above. The aromatic vinyl polymer block chains produced through polymerization usually have an active terminal. The resulting aromatic vinyl polymer block chains will form the aromatic vinyl polymer block (Ar1$^a$) or the aromatic vinyl polymer block (Ar1$^b$). For this reason, the amount of the monomers used in the first polymerization step may be determined according to the weight average molecular weight of the aromatic vinyl polymer block (Ar1$^a$) or the aromatic vinyl polymer block (Ar1$^b$).

In the next step, the monomers containing a conjugated diene monomer as the main component are added to the solution containing aromatic vinyl polymer block chains prepared in the first polymerization step, and polymerization is performed (second polymerization step). Thereby, a solution containing diblock chains can be prepared. The diblock chains produced through polymerization usually have an active terminal. In the diblock chains produced through the second polymerization step, the polymer chain produced through the first polymerization step which will form the aromatic vinyl polymer block (Ar1$^a$) or the aromatic vinyl polymer block (Ar1$^b$) is bonded to the polymer chain which will form the conjugated diene polymer block (D$^a$) or the conjugated diene polymer block (D$^b$). For this reason, the amount of the monomers used in the second polymerization step may be determined according to the weight average molecular weight of the conjugated diene polymer block (D$^a$) or the conjugated diene polymer block (D$^b$). The temperature for the polymerization reaction, the polymerization time, the polymerization pressure, and the condition for stirring the polymerization reaction system may be controlled within the same ranges as those in the first polymerization step.

From the viewpoint of the capability of properly controlling the molecular weight distribution (Mw/Mn) of the obtained block copolymer to fall within the above ranges, in the conjugated diene monomer used in the polymerization, the amount of deactivation components, such as water, which deactivate the polymerization initiator, is preferably controlled to be 1 × 10$^{-3}$ mol/kg or less. In the following conjugated diene monomer used in the production of each block copolymer, from the viewpoint of properly controlling the molecular weight distribution (Mw/Mn) of the obtained block copolymer to fall within the above ranges, the amount of deactivation components is preferably controlled to be within the above range.

In the next step, the monomers containing an aromatic vinyl monomer as the main component are added to the solution containing diblock chain prepared in the second polymerization step, and polymerization is performed (third polymerization step). Thus, a solution containing triblock chains can be obtained. Note that the triblock chains obtained by polymerization usually have an active terminal. Further, in the triblock chains produced in the third polymerization step, the polymer chain produced in the second polymerization step which will form the aromatic vinyl polymer block (Ar1$^a$) or the aromatic vinyl polymer block (Ar1$^b$) and the conjugated diene polymer block (D$^a$) or the conjugated diene polymer block (D$^b$) is further bonded to the polymer chain which will form a part of the aromatic vinyl polymer block (Ar2$^a$) or the aromatic vinyl polymer block (Ar2$^b$). For this reason, the amount of the monomers used in the third polymerization step may be determined according to the weight average molecular weight of the aromatic vinyl polymer block (Ar2$^b$). The temperature for the polymerization reaction, the polymerization time, the polymerization pressure, and the condition for stirring the polymerization reaction system may be controlled within the same ranges as those in the first polymerization step.

Then, a polymerization terminator is added to the solution containing the triblock chain obtained in the third polymerization step in an amount which becomes less than 1 molar equivalents with respect to the active terminals in the triblock chains (termination step). Thus, part of the active terminals in the triblock chains is deactivated to obtain a block copolymer in which the active terminals are deactivated. This block copolymer is a block copolymer B represented by the general formula (B): Ar1$^b$-D$^b$-Ar2$^b$.

The polymerization terminator is not particularly limited as long as it can react with and deactivate an active terminal and no longer reacts with other active terminals after reacting with one active terminal, but is preferably a polymerization terminator which is a compound which does not contain a halogen atom from the viewpoint of suppressing moisture absorption of the obtained copolymer, and among them, a polymerization terminator which generates a metal alkoxide, a metal aryl oxide, or a metal hydroxide when reacted with the active terminal is particularly preferred. Examples of the compound particularly preferably used as the polymerization terminator include water, monohydric alcohols such as methanol and ethanol, and monohydric phenols such as phenol and cresol.

The amount of the polymerization terminator to be used is determined according to the weight ratio of the block copolymer A and the block copolymer B to be contained in the aromatic vinyl block copolymer composition and is not particularly limited as long as it is an amount which becomes less than 1 molar equivalents with respect to the active terminals of the triblock chains, but is usually in a range in which the polymerization terminator becomes 0.18 to 0.91 molar equivalents with respect to the active terminals, preferably in a range in which the polymerization terminator becomes 0.35 to 0.80 molar equivalents.

As described above, when a polymerization terminator is added to the solution containing triblock chains having active terminals in an amount of less than 1 molar equivalents with respect to the active terminals thereof, part of the active terminals of the triblock chains having active terminals is deactivated, and a polymer in which its active terminals have been deactivated is obtained as a block copolymer B. Then, a portion of the triblock chains having active terminals which have not reacted with the polymerization terminator remains unreacted in the solution.

Then, monomers containing an aromatic vinyl monomer as the main component are added to the solution containing triblock chains and the block copolymer B obtained in the termination step, and polymerization is carried out (the fourth polymerization step). As a result, it is possible to obtain a solution containing the block copolymer A represented by the general formula (A): Ar1$^a$-D$^a$-Ar2$^a$ and the block copolymer B. In the block copolymer A produced in the fourth polymerization step, a polymer chain which forms a part of an aromatic vinyl polymer block (Ar2$^a$) is further bonded to the active terminal of the triblock chain obtained in the third polymerization step. For this reason, the amount of the monomers used in the fourth polymerization step may be determined according to the weight average molecular weight of the aromatic vinyl polymer block (Ar2$^a$). The temperature for the polymerization reaction, the polymerization time, the polymerization pressure, and the condition for stirring the polymerization reaction system may be controlled within the same ranges as those in the first polymerization step.

After the fourth polymerization step, the polymer component may be recovered from the solution containing the block copolymer A and the block copolymer B (recovery step). The method of recovery may be according to a conventional method, and is not particularly limited. For example, after completion of the reaction, if necessary, a polymerization terminator such as water, methanol, ethanol, propanol, hydrochloric acid, or citric acid is added, and if necessary, an additive such as an antioxidant is further added, and then a known method such as drying or steam stripping is applied directly to the solution to be recovered. When the polymer component is recovered as a slurry by applying steam stripping or the like, the polymer component may be dehydrated using an optional dehydrator such as an extruder type squeezer to obtain crumbs having a moisture content of a predetermined value or less, and the crumbs may be dried using an optional dryer such as a band dryer, an expansion extruder, and a twin screw extrusion dryer. The block copolymer obtained as described above may be processed into a pellet shape or the like according to a conventional method and then used.

As described above, the block copolymer A and the block copolymer B can be produced.

The block copolymer C and the block copolymer B are preferably produced by a method in which a polymer block is formed by sequentially polymerizing an aromatic vinyl monomer, a conjugated diene monomer, and then an aromatic vinyl monomer in this order.

More particularly, the block copolymer C is preferably produced by the following production method. In other words, preferred is a production method comprising:

a first polymerization step of obtaining a solution containing aromatic vinyl polymer block chains by polymerizing monomers containing an aromatic vinyl monomer in a solvent in the presence of a polymerization initiator,
a second polymerization step of obtaining a solution containing diblock chains by adding monomers containing a conjugated diene monomer to the solution containing aromatic vinyl polymer block chains, and performing polymerization, and
a third polymerization step of obtaining a solution containing the block copolymer C by adding monomers containing an aromatic vinyl monomer to the solution containing diblock chains, and performing polymerization.

In the above production method, first, monomers containing an aromatic vinyl monomer as the main component are polymerized using a polymerization initiator in a solvent (first polymerization step). The solvent used in the first polymerization step and the amount thereof used and the polymerization initiator and the amount thereof used may be the same as those in the first polymerization step of the above-described preferred production method of the block copolymer A and the block copolymer B. In addition, a Lewis base compound may be added in the same manner as in the first polymerization step of the above-described preferred production method of the block copolymer A and the block copolymer B, and the amount thereof used may be the same as that in the first polymerization step. Further, the same polymerization reaction temperature, polymerization time, polymerization pressure, and condition for stirring the polymerization reaction system as those in the first polymerization step of the above-described preferred production method of the block copolymer A and the block copolymer B may be employed.

Under the above conditions, a solution containing aromatic vinyl polymer block chains can be obtained by polymerizing the monomers containing the aromatic vinyl monomer as the main component using the polymerization initiator in the solvent. Note that the aromatic vinyl polymer block chains obtained by polymerization usually have an active terminal. The obtained aromatic vinyl polymer block chain will form the aromatic vinyl polymer block ($Ar1^c$). For this reason, the amount of the monomers used in this first polymerization step may be determined according to the weight average molecular weight of the aromatic vinyl polymer block ($Ar^c$).

Then, monomers containing a conjugated diene monomer as the main component are added to the solution containing aromatic vinyl polymer block chains obtained in the first polymerization step, and polymerization is carried out (second polymerization step). Thus, a solution containing diblock chains can be obtained. Note that the diblock chains obtained by polymerization usually have an active terminal. In addition, in the diblock chains produced through the second polymerization step, the polymer chain produced through the first polymerization step which will form the aromatic vinyl polymer block ($Ar1^c$) is bonded to the polymer chain which will form the conjugated diene polymer block ($D^c$). For this reason, the amount of the monomers used in the second polymerization step may be determined according to the weight average molecular weight of the conjugated diene polymer block ($D^c$). The temperature for the polymerization reaction, the polymerization time, the polymerization pressure, and the condition for stirring the polymerization reaction system may be controlled within the same ranges as those in the first polymerization step.

In the next step, monomers containing an aromatic vinyl monomer as the main component are added to the solution containing diblock chains prepared in the second polymerization step, and polymerization is performed (third polymerization step). Thus, a solution containing the block copolymer C represented by the general formula (C), $Ar1^c$-$D^c$-$Ar2^c$, can be obtained. In the block copolymer C produced in the third polymerization step, the polymer chain produced in the second polymerization step which will form the aromatic vinyl polymer block ($Ar1^c$) and the conjugated diene polymer block ($D^c$) is further bonded to the polymer chain which will form the aromatic vinyl polymer block ($Ar2^c$). For this reason, the amount of the monomers used in the third polymerization step may be determined according to the weight average molecular weight of the aromatic vinyl polymer block ($Ar2^c$). The temperature for the polymerization reaction, the polymerization time, the polymerization pressure, and the condition for stirring the polymerization reaction system may be controlled within the same ranges as those in the first polymerization step.

After the third polymerization step, the polymer component may be recovered from the solution containing the block copolymer C and the block copolymer B (recovery step). The method of recovery may be according to a conventional method, and is not particularly limited. For example, after completion of the reaction, if necessary, a polymerization terminator such as water, methanol, ethanol, propanol, hydrochloric acid, or citric acid is added, and if necessary, an additive such as an antioxidant is further added, and then a known method such as drying or steam stripping is applied directly to the solution to be recovered. When the polymer component is recovered as a slurry by applying steam stripping or the like, the polymer component may be dehydrated using an optional dehydrator such as an extruder type squeezer to obtain crumbs having a moisture content of a predetermined value or less, and the crumbs may be dried using an optional dryer such as a band dryer, an expansion extruder, and a twin screw extrusion dryer. The block copolymer obtained as described above may be processed into a pellet shape or the like according to a conventional method and then used.

As described above, the block copolymer A and the block copolymer C can be produced.

It is preferable that the block copolymer D be produced by a method in which an aromatic vinyl monomer and a conjugated diene monomer are polymerized to form diblock chains, and the diblock chains and a coupling agent are reacted to perform coupling.

More particularly, the block copolymer D is preferably produced by the following production method. That is a production method comprising,

a first polymerization step of obtaining a solution containing aromatic vinyl polymer block chains by polymerizing monomers containing an aromatic vinyl monomer in a solvent in the presence of a polymerization initiator,
a second polymerization step of obtaining a solution containing diblock chains by adding monomers containing a conjugated diene monomer to the solution containing aromatic vinyl polymer block chains, and performing polymerization,
a reaction step of adding a coupling agent to the solution containing diblock chains to obtain a block copolymer D.

In the above production method, first, the monomers containing an aromatic vinyl monomer as the main component are polymerized using a polymerization initiator in a solvent (first polymerization step). The solvent used in the first polymerization step and the amount thereof used and the polymerization initiator and the amount thereof used may be the same as those in the first polymerization step of the above-described preferred production method of the block copolymer A and the block copolymer B. In addition, a Lewis base compound may be added in the same manner as in the first polymerization step of the above-described preferred production method of the block copolymer A and the block copolymer B, and the amount thereof used may be the same as that in the first polymerization step. Further, the same polymerization reaction temperature, polymerization time, polymerization pressure, and condition for stirring the polymerization reaction system as those in the first polymerization step of the above-described preferred production method of the block copolymer A and the block copolymer B may be employed.

Under the above conditions, a solution containing aromatic vinyl polymer block chains can be obtained by polymerizing the monomers containing an aromatic vinyl monomer as the main component using the polymerization initiator in the solvent. Note that the aromatic vinyl polymer block chains obtained by polymerization usually have an active terminal. The obtained aromatic vinyl polymer block chain will form the aromatic vinyl polymer block ($Ar^d$). For this reason, the amount of the monomers used in this first polymerization step may be determined according to the weight average molecular weight of the aromatic vinyl polymer block ($Ar^d$).

Then, a monomer containing a conjugated diene monomer as the main component is added to the solution containing aromatic vinyl polymer block chains obtained in the first polymerization step, and polymerization is carried out (second polymerization step). Thus, a solution containing diblock chains can be obtained. Note that the diblock chains obtained by polymerization usually have an active terminal. In addition, in the diblock chain produced through the second polymerization step, the polymer chain produced through the first polymerization step which will form the aromatic vinyl polymer block ($Ar^d$) is bonded to the polymer chain which will form the conjugated diene polymer block ($D^d$). For this reason, the amount of the monomers used in the second polymerization step may be determined according to the weight average molecular weight of the conjugated diene polymer block ($D^d$). The temperature for the polymerization reaction, the polymerization time, the polymerization pressure, and the condition for stirring the polymerization reaction system may be controlled within the same ranges as those in the first polymerization step.

Then, to the solution containing diblock chains, a coupling agent is added (reaction step). Thus, the active terminals of the diblock chains and the coupling agent react with each other, so that two or more diblock chains are bonded via residues of the coupling agent to form the block copolymer D.

The coupling agent is not particularly limited as long as it has two or more functional groups in one molecule capable of reacting with the active terminal of the diblock chain, but a coupling agent having 2 to 8 functional groups in one molecule capable of reacting with the active terminal of the diblock chain is preferred, a coupling agent having 2 to 4 functional groups in one molecule capable of reacting with the active terminal of the diblock chain is more preferred. Further, the coupling agent preferably contains a silicon atom. More preferred are halogenated silanes and alkoxysilanes.

As a coupling agent having two functional groups in one molecule capable of reacting with the active terminal of the diblock chain (two functional coupling agent), for example, two functional halogenated silanes such as dichlorosilane, monomethyldichlorosilane, and dimethyldichlorosilane; two functional alkoxysilanes such as diphenyldimethoxysilane and diphenyldiethoxysilane; two functional halogenated alkanes such as dibromoethane, methylene chloride, and dibromomethane; two functional tin halides such as dichlorotin, monomethyldichlorotin, dimethyldichlorotin, monoethyldichlorotin, monobutyldichlorotin, and dibutyldichlorotin; dibromobenzene, benzoic acid, CO, 2-chloropropene, and the like may be used. Among these, two functional halogenated silanes or two functional alkoxysilanes are particularly preferably used. These two functional coupling agents may be used alone or in combination.

Examples of a coupling agent having three functional groups in one molecule capable of reacting with the active terminal of the diblock chain (three functional coupling agent) include three functional halogenated alkanes such as trichloroethane and trichloropropane; three functional halogenated silanes such methyltrichlorosilane and ethyltrichlorosilane; three functional alkoxysilanes such as methyltrimethoxysilane, phenyltrimethoxysilane, and phenyltriethoxysilane; and the like. These three functional coupling agents may be used alone or in combination.

Examples of a coupling agent having four functional groups in one molecule capable of reacting with the active terminal of the diblock chain (four functional coupling agent) include four functional halogenated alkanes such as carbon tetrachloride, carbon tetrabromide, and tetrachloroethane; four functional halogenated silanes such as tetrachlorosilane and tetrabromosilane; four functional alkoxysilanes such as tetramethoxysilane and tetraethoxysilane; four functional tin halides such as tetrachlorotin and tetrabromotin; and the like. These four functional coupling agents may be used alone or in combination.

The number of "n" of the block copolymer D, that is, the number of branches of the block copolymer D can be controlled by adjusting the type of the coupling agent, the amount thereof used, the timing of the addition thereof, the amount of the Lewis base compound used, and the like. Also, a reaction terminator such as methanol can be used to adjust the coupling ratio to adjust the number of branches of the block copolymer D. Further, by using two or more kinds of coupling agents having different numbers of functional groups capable of reacting with the active terminal in the diblock chain in combination, it is also possible to adjust the number of branches of the block copolymer D. Further, by these means, the diblock chains produced in the second polymerization step may be left unreacted, and finally recovered as the block copolymer E.

The amount of the coupling agent to be used is adjusted to an appropriate amount according to the number of branches of the target block copolymer D. The amount of the coupling agent to be used is preferably 0.05 to 1.0 molar equivalents, more preferably 0.50 to 1.0 molar equivalents, still more preferably 0.85 to 1.0 molar equivalents with respect to the active terminals of the diblock chains.

The reaction temperature is preferably 10 to 100°C, more preferably 20 to 90°C, still more preferably 30 to 80°C. The time required for the reaction varies depending on the conditions, but is usually within 48 hours, preferably 0.05 to 2 hours, still more preferably 0.1 to 1.0 hours.

After the reaction step, the polymer component may be recovered from the solution containing the block copolymer D and the block copolymer E (recovery step). The method of recovery may be according to a conventional method, and is not particularly limited. For example, after completion of the reaction, if necessary, a polymerization terminator such as water, methanol, ethanol, propanol, hydrochloric acid, or citric acid is added, and if necessary, an additive such as an antioxidant is further added, and then a known method such as drying or steam stripping is applied directly to the solution to be recovered. When the polymer component is recovered as a slurry by applying steam stripping or the like, the polymer component may be dehydrated using an optional dehydrator such as an extruder type squeezer to obtain crumbs having a moisture content of a predetermined value or less, and the crumbs may be dried using a dryer such as a band dryer, an expansion extruder, and a twin screw extrusion dryer. The block copolymer obtained as described above may be processed into a pellet shape or the like according to a conventional method, and then used.

As described above, the block copolymer D and optionally the block copolymer E can be produced.

The aromatic vinyl block copolymer composition used in the present invention may further contain, if necessary, a fatty acid amide, a polyethylene wax, an antioxidant, a tackifying resin, a softening agent, an antibacterial agent, a light stabilizer, an ultraviolet absorber, a dye, a lubricant, and the like.

The fatty acid amide may be an aliphatic monoamide or an aliphatic bisamide. The aliphatic monoamide is not particularly limited as long as it is a compound obtained by bonding a hydrocarbon group and one amide group (-NHCO), but a monoamide of a higher saturated fatty acid having 12 or more carbon atoms (that is, a compound obtained by bonding a chain alkyl group having 12 or more carbon atoms and a one amide group (-NHCO)) is preferably used.

Specific examples of fatty acid monoamides include saturated fatty acid monoamides such as lauric amide, myristic amide, palmitic amide, stearic amide, behenic amide, and hydroxystearic amide; unsaturated fatty acid monoamide such as oleic amide and erucic amide; and the like.

The content of the fatty acid amide is preferably 0.2 to 10 parts by weight, more preferably 0.3 to 8 parts by weight, still more preferably from 0.5 to 6 parts by weight with respect to 100 parts by weight of the total of the block copolymer A, the block copolymer B, at least one selected from the group consisting of the block copolymer C and the block copolymer D, and optionally the block copolymer E, if contained.

The polyethylene wax is a wax having an ethylene monomer unit as the main constitutional unit. The polyethylene wax used in the present invention is not particularly limited, but preferably used is the one having a viscosity of 20 to 6,000 mPa·s at 140°C.

The polyethylene waxes are generally produced by polymerization of ethylene or decomposition of polyethylene, but in the present invention, either type of polyethylene wax may be used. In addition, polyethylene wax is available in commercially available products, and specific examples thereof include "A-C polyethylene" (manufactured by Honeywell), "Mitsui High Wax" (manufactured by Mitsui Chemical Incorporatied), "Sanwax" (manufactured by Sanyo Chemical Industries, Ltd.), and "epolene" (manufactured by Eastman Chemical Company).

The antioxidant is not particularly limited, and for example, hindered phenolic compounds such as pentaerythritol tetrakis[3-(3,5-dit-butyl-4-hydroxyphenyl)propionate], octadecyl-3-(3,5-di-t-butyl-4-hydroxyphenyl)propionate, 2,6-di-t-butyl-p-cresol, di-t-butyl-4-methylphenol, 4-[[4,6-bis(octylthio)-1,3,5-triazin-2-yl]amino]-2,6-di-tert-butylphenol, 2,4-bis[(dodecylthio)methyl]-6-methylphenol, and 4,6-bis(octylmethyl)-o-cresol; thiodicarboxylate esters such as dilauryl thiopropionate; and phosphites such as tris(nonylphenyl)phosphite and butylidenebis(3-methyl-6-t-butylphenyl-di-tridecyl phosphite may be used.

The content of the antioxidant is preferably 10 parts by weight or less, more preferably 0.05 to 5 parts by weight with respect to 100 parts by weight of the total of the block copolymer A, the block copolymer B, at least one selected from the group consisting of the block copolymer C and the block copolymer D, and optionally the block copolymer E, if contained.

The aromatic vinyl block copolymer composition used in the present invention may further contain a wax containing a linear hydrocarbon and a branched hydrocarbon. The wax used in the present invention may be a natural wax or a synthetic wax, but is preferably a natural wax, and more preferably a petroleum wax.

A blended wax in which a petroleum wax is mainly contained and additional natural wax and synthetic wax are partially mixed therewith may be used. Further, for the purpose of improving the function and workability of these waxes, a small amount of resin or the like may be further blended with the wax.

The linear hydrocarbon is preferably a linear aliphatic saturated hydrocarbon. The linear hydrocarbon is sometimes referred to as normal paraffin. The branched hydrocarbon is preferably a branched aliphatic saturated hydrocarbon. The branched hydrocarbon is sometimes called isoparaffin. As the linear hydrocarbon and the branched hydrocarbon, a linear hydrocarbon and a branched hydrocarbon contained in a petroleum wax are preferred.

In addition to the linear hydrocarbon and the branched hydrocarbon, a cyclic hydrocarbon may be contained in the wax used in the present invention, but the total amount of the linear hydrocarbon and the branched hydrocarbon in the wax is preferably 50% by mass or more, more preferably 70% by mass or more, still more preferably 80% by mass or more, most preferably 90% by mass or more.

The weight ratio (n-structure/iso-structure) of the linear hydrocarbon (n-structure) and the branched hydrocarbon (iso-structure) in the wax is 30/70 to 99/1, preferably 50/50 to 97/3, more preferably 70/30 to 95/5, most preferably 75/25 to 90/10.

The melting point of the wax is less than 80°C, preferably less than or equal to 76°C, more preferably less than or equal to 73°C, preferably greater than or equal to 30°C, more preferably greater than or equal to 40°C, still more preferably greater than or equal to 45°C. The melting point of the wax can be measured by using a differential scanning calorimeter or the like. If the melting point of the wax is too high, it becomes difficult to obtain an elastic body having excellent ozone crack resistance. If the melting point of the wax is too low, it is easy to block during transportation and storage, and it is easy to melt during processing, resulting in poor handling properties.

The content of the wax in the aromatic vinyl block copolymer composition used in the present invention is preferably 0.1 to 5 parts by weight, more preferably 0.3 to 3.5 parts by weight, still more preferably 0.5 to 2 parts by weight with respect to 100 parts by weight of the total of the block copolymer A, the block copolymer B, at least one selected from the group consisting of the block copolymer C and the block copolymer D, and optionally the block copolymer E, if contained.

In obtaining the aromatic vinyl block copolymer composition used in the present invention, a method of mixing the block copolymer A, the block copolymer B, at least one selected from the group consisting of the block copolymer C and the block copolymer D, and optionally the block copolymer E, if contained, with other components is not particularly limited. Examples thereof include a method in which the components are dissolved in a solvent and uniformly mixed, and then the solvent is removed by heating or the like, and a method in which the components are melt-mixed by a screw extruder, a kneader, or the like. Among these, melt mixing is suitable from the viewpoint of more efficiently performing mixing. Note that the temperature at the time of performing melt mixing is not particularly limited, but is usually in the range of 100 to 250°C.

When the block copolymer A, the block copolymer B, at least one selected from the group consisting of the block copolymer C and the block copolymer D, and optionally the block copolymer E, if contained, and other components are melt-mixed, all or part of the components of one or more of the block copolymer A, the block copolymer B, at least one selected from the group consisting of the block copolymer C and the block copolymer D, and optionally the block copolymer E, if contained, and other components may be kneaded in advance by a single screw extruder, a twin screw extruder, a Banbury mixer, a kneader, or the like to prepare a master batch pellet in order to more uniformly mix them. Among the above, it is preferable to use a twin screw extruder or a Banbury mixer in particular in view of kneadability, cleaning properties, and cost.

In the above description, an aromatic vinyl block copolymer composition comprising the block copolymer A, the block copolymer B, at least one selected from the group consisting of the block copolymer C and the block copolymer D, and optionally the block copolymer E, if contained, has been described as one example. The thermoplastic elastomer according to the present invention is not particularly limited to such an aromatic vinyl block copolymer composition, but it is sufficient that it is a thermoplastic elastomer which satisfies all of the above-described (1) to (4). When all of the above-described (1) to (4) are satisfied, a film-shaped elastic body producing a high stress and having a high stretchability and a large width can be provided. It is sufficient that the thermoplastic elastomer according to the present invention satisfies all of the above-described (1) to (4), and the chemical composition, the shape, the size, and the like are not particularly limited. The thermoplastic elastomer according to the present invention may have any shape, and may be film-shaped or pellet-shaped. The film-shaped thermoplastic elastomer may have a plurality of pores to impart air-permeability.

The thermoplastic elastomer according to the present invention may contain a polyolefin elastomer in addition to the aromatic vinyl block copolymer, and preferably contains the aromatic vinyl block copolymer in a proportion of more than 50% by weight, and contains a polyolefin elastomer in a proportion of more than 1% by weight and less than 50% by weight. When a polyolefin elastomer is contained in such an amount, the stress relaxation property can be improved.

In particular, a stretchable film used in hygienic products such as paper diapers and sanitary products is required to have high moisture permeability from the viewpoint of prevention from getting sweaty. Examples of a method for achieving high moisture permeability include a method of using a stretchable film having a plurality of pores. In this case, when the aperture ratio of the film is increased, the moisture permeability is improved. On the other hand, when the aperture ratio of the film is increased, the area of the actual film portion decreases, and thus the actual film portion may receive excessive stress. Consequently, the film can tear from the pores during use, or a phenomenon in which a stretchable member cannot recover its original length may occur due to stress relaxation of the material itself (this phenomenon is called "loss of resilience". "Loss of resilience" causes inferior fitting to the wearer's body, and leakage of excreta due to a space created between the wearer's body and the diaper. On the other hand, when a polyolefin elastomer is contained in the amount described above, the stress relaxation property can be improved. Thus, even when the aperture ratio of the film is increased, these troubles can be effectively avoided.

The content of the polyolefin elastomer in the thermoplastic elastomer according to the present invention is preferably more than 1% by weight and less than 50% by weight, more preferably 3% by weight or more and less than 25% by weight, still more preferably 5 to 17% by weight, even more preferably 7 to 13% by weight. The content of the aromatic vinyl block copolymer in the thermoplastic elastomer according to the present invention is preferably more than 50% by weight, more preferably more than 75% by weight and 97% by weight or less, still more preferably 83 to 95% by weight, even more preferably 87 to 93% by weight. When the content of polyolefin elastomer is within the above ranges, the stress relaxation property can be further improved while the high stress and the small permanent tension set is maintained. In this case, the aromatic vinyl block copolymer composition described above can be suitably used as the aromatic vinyl block copolymer.

The polyolefin elastomer is not particularly limited as long as it is a resin having thermoplasticity containing an olefin as the main repeating unit, and may be any of a homopolymer of an α-olefin, a copolymer of two or more α-olefins, and a copolymer of an α-olefin and a monomer other than the α-olefin, and may be a polymer obtained by modifying these (co)polymers.

Specific examples of the polyolefin elastomer include homopolymers or copolymers of an α-olefin such as ethylene and propylene. Examples thereof include homopolymers of α-olefins, such as polyethylenes such as linear low-density polyethylene (LLDPE), low-density polyethylene (LDPE), medium-density polyethylene (MDPE), high-density polyethylene (HDPE), metallocene polyethylene, polypropylene, metallocene polypropylene, polymethylpentene, and polybutene; copolymers of ethylene with other α-olefins, such as ethylene-propylene random copolymer, ethylene-propylene block copolymers, and ethylene-butene-1 copolymers, ethylene-propylene-butene-1 copolymer, ethylene-octene copolymer, and ethylene-cycloolefin copolymer; copolymers of α-olefins with carboxylic unsaturated alcohols which mainly contain α-olefins and saponified products thereof, such as ethylene-vinyl acetate copolymer and ethylene-vinyl alcohol copolymer; copolymers of an α-olefin and an α,β-unsaturated carboxylic acid ester or an α,β-unsaturated carboxylic acid and the like which contain α-olefins, such as ethylene-α,β-unsaturated carboxylic acid ester copolymers (ethylene-ethyl acrylate copolymer, ethylene-ethyl methacrylate copolymer, and the like) and ethylene-α,β-unsaturated carboxylic acid copolymers (ethylene-acrylic acid copolymer, ethylene-methacrylic acid copolymer, and the like); acid-modified olefin resins such as α-olefin (co)polymers, such as polyethylene and polypropylene, which are modified by an unsaturated carboxylic acids (such as acrylic acid, methacrylic acid, maleic acid, maleic anhydride, fumaric acid, or itaconic acid) and/or an anhydride thereof; ionomer resins in which Na ion, Zn ion, and the like are brought into action on copolymers of ethylene and methacrylic acid; and mixtures thereof. Among these, the polyolefin elastomer preferably contains an ethylene unit, and is more preferably polyethylene or a copolymer of ethylene and other α-olefin, still more preferably a copolymer of ethylene and propylene, particularly preferably a block copolymer of ethylene and propylene.

The weight average molecular weight of the polyolefin elastomer is not particularly limited, but is usually selected in the range of 10,000 to 5,000,000, and is preferably selected in the range of 50,000 to 800,000. The specific gravity and the melt index of the polyolefin elastomer are not particularly limited, but the specific gravity is usually selected in the range of 0.80 to 0.95 g/cm$^3$, preferably in the range of 0.85 to 0.94 g/cm$^3$, and the melt index is usually selected in the range of 0.1 to 1,000 g/10 min, preferably in the range of 1 to 500 g/10 min, more preferably in the range of 2 to 15 g/10 min, as values measured in accordance with ASTM D-1238 (G condition, 200°C, 5 Kg).

The thermoplastic elastomer according to the present invention can be used, for example, in films, yarns (elastic strands), gloves, elastic bands, condoms, OA equipment, rolls for office equipment, and the like, forming materials used for vibration-proof sheets for electrical and electronic equipment, vibration-proof rubbers, shock absorbing sheets, shock cushioning films or sheets, residential vibration-proof sheets, vibration-damper materials, and the like, adhesive applications used for adhesive tapes, adhesive sheets, adhesive labels, various removing rollers, and the like, adhesive applications used for hygienic products and bookbinding, elastic fiber applications used for clothing, sports products, and the like.

The thermoplastic elastomer according to the present invention can be suitable used in a film, and in particular, suitably used in a stretchable film for use in hygienic products such as paper diapers and sanitary products (film-shaped stretchable members for use in hygienic materials).

The thickness of the film according to the present invention is not particularly limited, and is adjusted depending on the application, and in the application to a film for use in hygienic products such as paper diapers and sanitary products, the thickness is preferably 0.01 to 5 mm, more preferably 0.01 to 1 mm, still more preferably 0.02 to 0.2 mm, most preferably 0.02 to 0.06 mm. The film can be suitably used as a porous film (film-shaped porous body) for use in hygienic products such as paper diapers and sanitary products when the thermoplastic elastomer according to the present invention contains the aromatic vinyl block copolymer in a proportion of more than 50% by weight and contains a polyolefin elastomer in a proportion of more than 1% by weight and less than 50% by weight. In this case, when the aperture ratio is increased, the film can have excellent moisture permeability and an excellent stress relaxation property.

The method of forming the thermoplastic elastomer of the present invention into a film is not particularly limited, and a conventionally known film forming method can be applied, but from the viewpoint of obtaining a smooth film with good productivity, melt extrusion molding is suitable, and especially, melt extrusion molding using a T-die is particularly suitable.

The method for producing a stretchable laminate having a layered configuration composed of nonwoven fabric/stretchable film/nonwoven fabric by laminating a stretchable film for use in hygienic products such as paper diapers and sanitary products formed from the thermoplastic elastomer according to the present invention onto nonwoven fabrics as substrate sheets such that the stretchable film is interposed therebetween is exemplified, and described below.

Fig. 1 shows a schematic illustration of a production apparatus for producing a stretchable laminate according to one embodiment of the present invention. As shown in Fig. 1, the stretchable laminate production apparatus comprises a melt extrusion mechanism 10, a cooling roll 11, a stretching mechanism 20, a lamination mechanism 30, and a controller (not shown). The controller controls the melt extrusion mechanism 10, the cooling roll 11, the stretching mechanism 20, and the lamination mechanism 30 to operate cooperatively.

The melt extrusion mechanism 10 discharges a heated and melted thermoplastic elastomer into a film shape to form a melted material 40 that is film- or line-shaped. The melt extrusion mechanism 10 is, for example, a melt extrusion molding mechanism using a T-die, and may be a twin screw extruder equipped with a T-die.

As shown in Fig. 1, the cooling roll 11 is positioned under the melt extrusion mechanism 10, and the melted material 40 discharged from the melt extrusion mechanism 10 is stretched until it reaches the cooling roll 11. That is, the cooling roll 11 revolves at a peripheral speed faster than the feeding speed of the thermoplastic elastomer discharged from the discharge port of the melt extrusion mechanism 10, and thus the melted material 40 is stretched until the thickness of the film-shaped melted material 40 becomes a predetermined thickness. In the present invention, from the viewpoint of broadening the width of the melted material 40 discharged from the melt extrusion mechanism 10 to finally provide a film having a larger width, the distance between the discharge port of the melt extrusion mechanism 10 and the cooling roll 11 is preferably as short as possible, and is preferably less than 50 mm. In addition, from the viewpoint of allowing the melted material 40 discharged from the melt extrusion mechanism 10 to come into contact with the cooling roll 11 as soon as possible, it is also preferable to provide a blowing mechanism for blowing air on the melted material 40 (i.e., a mechanism that blows air flowing in the left-to-right direction in the illustration on the melted material 40 discharged from the discharge port of the melt extrusion mechanism 10).

The cooling roll 11 has an internal flow path (not shown) through which a coolant flows, and cools the melted material 40 to a temperature range at which the thermoplastic elastomer constituting the melted material 40 experiences elastic deformation and solidifies the melted material 40 while the melted material 40 travels in the feeding direction in contact with the outer surface of the cooling roll 11. Consequently, a stretchable film 40a is formed from the melted material 40 and drawn from the cooling roll 11.

The stretchable film 40a is delivered to the stretching mechanism 20 through a guide roll 12. The stretching mechanism 20 comprises a drawing roll 13 and a drawing nip roll 14 facing the drawing roll, a first stretching roll 15 and a stretching nip roll 16 facing the first stretching roll, and a second stretching roll 17.

The stretchable film 40a is stretched in a section where the stretchable film 40a is delivered from the area between the drawing roll 13 and the drawing nip roll 14 to the second stretching roll 17 through the area between the first stretching roll 15 and the stretching nip roll 16. That is, the drawing roll 13, the first stretching roll 15, and the second stretching roll 17 revolve at different peripheral speeds that increase in this order to stretch the stretchable film 40a at predetermined rates in two steps. Specifically, the stretchable film 40a is stretched between the drawing roll 13 and the first stretching roll 15 by the difference in peripheral speed (by the action of the first stretching roll 15 revolving at a faster peripheral speed) by a first stretch rate, and then the stretchable film 40a stretched by the first stretch rate is stretched between the first stretching roll 15 and the second stretching roll 17 by the difference in peripheral speed (by the action of the second stretching roll 17 revolving at a faster peripheral speed) by a second stretch rate.

The lamination mechanism 30 supplies a first nonwoven fabric 50 as a first substrate sheet to a second stretching roll 17 through a guide roll 18, and supplies a second nonwoven fabric 60 as a second substrate sheet to the second stretching roll 17 through a guide roll 19. Then, the stretchable film 40a stretched along the second stretching roll 17 is sandwiched between the first nonwoven fabric 50 and the second nonwoven fabric 60 to form a stretchable laminate 70 in which the stretchable film 40a is laminated between the first nonwoven fabric 50 and the second nonwoven fabric 60. The resulting stretchable laminate 70 may be subjected to ultrasonic welding or heat welding by heat, if necessary. Fig. 1 exemplifies a case where ultrasonic welding is carried out using an ultrasonic welder 80. In the case where the stretchable laminate 70 is a film-shaped porous body, when ultrasonic welding is carried out, patterned openings can be formed by the application of ultrasonic using the ultrasonic welder 80.

As described above, the stretchable laminate 70 can be produced using a production apparatus shown in Fig. 1. When produced as described above, the stretchable laminate 70 has stretchability at least in the MD direction.

The thermoplastic elastomer according to the present invention satisfies all of the following (1) to (4):

(1) The storage modulus at 23°C is more than 0.3 MPa.

(2) The loss tangent ($\tan\delta$) at 23°C is 0.20 or less.

(3) When the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5.

(4) When the thermoplastic elastomer is extruded into a film shape by melt extrusion using a melt extrusion apparatus, and cooled and solidified to form a film and the film is stretched in the MD direction, the film does not have a yield point at an elongation rate of 200% or less.

Thus, according to the thermoplastic elastomer according to the present invention, when production is carried out using the production apparatus shown in Fig. 1, a reduction in width after the film is discharged from the melt extrusion mechanism 10 can be prevented, and a reduction in width of the film by stretching (stretching in the MD direction) can be prevented. Thus, a stretchable film and a stretchable laminate producing a high stress and having a high stretchability and a large width can be provided.

EXAMPLES

Hereinafter, the present invention is more specifically described by way of Examples and Comparative Examples, but the present invention is not limited to the examples. The term "part(s)" refers to part(s) by weight unless otherwise indicated. Test methods used in Examples and Comparative Examples are as follows.

[Weight average molecular weights of block copolymers]

The weight average molecular weight of each of the block copolymers was determined as a molecular weight in terms of polystyrene by high performance liquid chromatography using tetrahydrofuran having a flow rate of 0.35 ml/min as a carrier. The device used was a HLC8320 available from Tosoh, three columns connected were Shodex KF-404HQ available from Showa Denko K. K. (column temperature: 40°C), a differential refractometer and an ultraviolet detector were used as detectors, and the molecular weight was calibrated at 12 points of standard polystyrenes (500 to 3 million) available from Tosoh.

[Weight ratio of block copolymers in block copolymer composition]

It was determined from the area ratios of the peaks corresponding to the block copolymers in the chart obtained by high performance liquid chromatography described above.

[Weight average molecular weight of styrene polymer block of each block copolymer]

According to the process described in Rubber Chem. Technol., 45, 1295 (1972), the block copolymer was reacted with ozone and reduced with lithium aluminum hydride to decompose the isoprene polymer block in the block copolymer. Specifically, the following procedure was carried out. That is, 300 mg of a sample was dissolved in a reaction vessel containing 100 ml of dichloromethane treated with molecular sieves. This reaction vessel was placed into a cooling tank, and cooled to -25°C, and ozone generated by an ozone generator was introduced while oxygen was flowed into the reaction vessel at a flow rate of 170 ml/min. After 30 minutes from the start of the reaction, it was confirmed that the reaction was completed by introducing the gas flowing out of the reaction vessel into the aqueous potassium iodide solution. Then, 50 ml of diethyl ether and 470 mg of lithium aluminum hydride were charged into another reaction vessel subjected to nitrogen substitution, and the solution reacted with ozone was slowly added dropwise to this reaction vessel while the reaction vessel with ice water was being cooled. Then, the reaction vessel was placed in a water bath, and the temperature was gradually increased to reflux at 40°C for 30 minutes. Thereafter, while the solution was being stirred, dilute hydrochloric acid was added dropwise in small portions to the reaction vessel, and the addition was continued until the generation of hydrogen was hardly recognized. After this reaction, the solid product formed in the solution was filtered off, and the solid product was extracted with 100 ml of diethyl ether for 10 minutes. The extract was combined with the filtrate after filtration, and the solvent was distilled off to obtain a solid sample. The sample thus obtained was measured for weight average molecular weight according to the method of measuring the weight average molecular weight described above, and the value thereof was defined as the weight average molecular weight of the styrene polymer block.

[Weight average molecular weight of isoprene polymer block in each block copolymer]

From the weight average molecular weight of each block copolymer determined as described above, the weight average molecular weight of the corresponding styrene polymer block was subtracted, and the weight average molecular weight of the isoprene polymer block was determined based on the calculated value.

[Cis/trans ratio of isoprene polymer block in each block copolymer]

It was determined based on the measurement by proton NMR.

[Styrene unit content of each block copolymer]

It was determined based on the detection intensity ratio between the differential refractometer and the ultraviolet detector in the measurement of the high performance liquid chromatography. Note that, in advance, copolymers having different styrene unit contents were prepared, and using them, a calibration curve was prepared.

[Storage modulus and loss tangent ($\tan\delta$)]

A thermoplastic elastomer was heat pressed at 170°C to prepare a sheet having a thickness of 2 mm. This sheet was punched out into a circular sheet having a diameter ($\varphi$) of 8 mm using a circular die having a diameter ($\varphi$) of 8 mm. The storage modulus G' at 23°C and the loss tangent ($\tan\delta$) were measured using a dynamic rheometer (trade name: ARES-G2 (available from TA instruments)). Parallel plates (a pair of two plates) having a diameter of 8 mm were used for the measurement. The measurement was carried out at a dynamic shear strain of 0.13% and at a frequency of 1 Hz (angular velocity: 6.28 rad/s$^{-1}$) . The measurement was carried out with heating from -40°C to 150°C at a heating rate of 4°C/min, and the data at 23°C was obtained.

[Ratio ($M_{MD}/M_{TD}$) between stress at 100% elongation in MD direction ($M_{MD}$) and stress at 100% elongation in TD direction ($M_{TD}$)]

The stress at 100% elongation of a film of a block copolymer composition was measured according to ISO 527-3 using a Tensilon universal testing machine RTC-1210 (available from ORIENTEC CO., LTD.).

· Shape of test piece: 25 mm wide × 90 mm long × 0.050 mm thick
· Testing temperature: 23°C
· Distance between clamps: 25 mm
· Testing speed: 300 mm/min

In this measurement, 2 pieces of the film were used. One of the pieces was measured along the melt flow direction (MD) of the molding, and the other piece was measured along the melt flow transverse direction (TD) of the molding, and the stress at 100% elongation in the MD direction ($M_{MD}$) and the stress at 100% elongation in the TD direction ($M_{TD}$) were determined to calculate their ratio ($M_{MD}/M_{TD}$).

[Yield point elongation in MD direction]

The yield point elongation of a film of a block copolymer composition was measured according to ISO 527-3 using a Tensilon universal testing machine RTC-1210 (available from ORIENTEC CO., LTD.) by stretching along the melt flow direction (MD) of the molding up to an elongation rate of 200%.

• Shape of test piece: 25 mm wide × 90 mm long × 0.050 mm thick
• Testing temperature: 23°C
• Distance between clamps: 25 mm
• Testing speed: 300 mm/min

[Stress at 200% elongation in MD direction]

The stress at 200% elongation of a film of a block copolymer composition in the melt flow direction (MD) of the molding was measured according to ISO 527-3 using a Tensilon universal testing machine RTC-1210 (available from ORIENTEC CO., LTD.), and the results were evaluated based on the following criteria.

○ : The stress at 200% elongation in the MD direction is 0.8 MPa or more
× : The stress at 200% elongation in the MD direction is less than 0.8 MPa

• Shape of test piece: 25 mm wide × 90 mm long × 0.050 mm thick
• Testing temperature: 23°C
• Distance between clamps: 25 mm
• Testing speed: 300 mm/min

[Permanent tension set in the MD direction]

The permanent tension set of a film of a block copolymer composition in the melt flow direction (MD) of the molding was measured using a Tensilon universal testing machine RTC-1210 (available from ORIENTEC CO., LTD.). Specifically, a test piece strip (i.e., strip-shaped sample) was used, the distance between marked lines was 25 mm before stretching, the test piece strip was stretched up to an elongation rate of 200% and held for 30 seconds in that state, and the test piece strip was allowed to recover the original length at the same speed. When the stress became zero while the test piece strip was allowed to recover the original length, the strain at the time was obtained, and the permanent tension set was calculated according to the following equation.

Permanent tension set (%) = (strain when stress becomes zero (mm)/distance between clamps (mm)) × 100

• Shape of test piece: 25 mm wide × 90 mm long × 0.050 mm thick
• Testing temperature: 23°C
• Distance between clamps: 25 mm
• Testing speed: 300 mm/min

The results were evaluated based on the following criteria.

∘ : The permanent tension set in the MD direction is 15% or less
× : The permanent tension set in the MD direction is more than 15%

[Film width]

Pellets of a thermoplastic elastomer were fed into a twin screw extruder equipped with a T-die. The pellets were heated and melted at 200°C, and kneaded. The kneaded thermoplastic elastomer was extruded so as to be sandwiched between release films made of PET to form a film having an average thickness of 0.05 mm. The resulting film was measured, and "the ratio of the width of the film/the width of the T-die" (= A) was calculated. The conditions for forming the film are detailed below.

(Conditions for forming films)

Thermoplastic elastomer processing rate: 5 kg/hour
Film drawing speed: 4 m/min
Extruder temperature: adjusted such that the inlet temperature was 100°C and the T-die temperature was 200°C
Screw: full flight & kneading
Extruder L/D: 30
T-die: 200 mm wide, 0.5 mm lip
Distance between tip of T-die and cooling roll: 2 mm
Cooling roll temperature: 23°C

Next, the film of the thermoplastic elastomer obtained above was stretched up to 200% in the melt flow direction (MD) of the molding and held, and immediately the width of the film in the stretched state was measured with reference to ISO 527-3 using a Tensilon universal testing machine RTC-1210 (available from ORIENTEC CO., LTD.), and "the width of the stretched film/the width of the film before stretching" (= B) was calculated and evaluated.

• Shape of test piece: 25 mm wide × 90 mm long × 0.050 mm thick
• Testing temperature: 23°C
• Distance between clamps: 25 mm

• Testing speed: 300 mm/min

The width of the film of the stretchable member is determined by the product (C) of the film width retention rate in the step of cooling and solidifying the film multiplied by the film width retention rate in the step of welding the stretched film onto the substrate sheet. When the value C is larger, it can be determined that a product having a large width can be more suitably obtained when such a film is used as a stretchable member for a stretchable film.

```
C: Ratio (%) of width of product to width of die = A × B × 100
```

∘: The ratio of the width of the product to that of the die is 60% or more.
×: The ratio of the width of the product to that of the die is less than 60%.

[Production Example 1]

To a pressure-resistant reactor, 23.3 kg of cyclohexane, 2.6 mmol of N,N,N',N'-tetramethylethylenediamine (hereinafter called "TMEDA"), and 0.91 kg of styrene were added, and stirred with a Reynolds number for stirring of 160,000 and a power used for rotating the stirring blade of 3 kW/m$^3$ at 40°C. To the mixture, 87.3 mmol of n-butyllithium was added while the stirring was continued, and polymerization was carried out for 1 hour with heating to 50°C and stirring continued under the same rotation conditions. The polymerization conversion of styrene was 100%. Subsequently, while the temperature was controlled to maintain 50 to 60°C and the stirring was continued under the same rotation conditions, 5.2 kg of isoprene was continuously added to the reactor over 1 hour. After the addition of the isoprene was completed, the polymerization was continued for another 1 hour with stirring continued under the same rotation conditions. The polymerization conversion of isoprene was 100%. Next, while the temperature was controlled to maintain 50 to 60°C and the stirring was continued under the same rotation conditions, 0.91 kg of styrene was continuously added over 1 hour. After the addition of the styrene was completed, the polymerization was continued for another 1 hour with stirring continued under the same rotation conditions to form triblock chains having active terminals. The polymerization conversion of styrene was 100%. Thereafter, 53.7 mmol of methanol was added as a polymerization terminator and mixed to deactivate part of the active terminals of the triblock chains having active terminals to form a styrene-isoprene-styrene triblock copolymer B1. Subsequently, while the temperature was controlled to maintain 50 to 60°C and the stirring was continued under the same rotation conditions, 2.97 kg of styrene was continuously added over 1 hour. After the addition of the styrene was completed, while the stirring was continued under the same rotation conditions, the polymerization was continued for another 1 hour to form triblock chains having active terminals. The polymerization conversion of styrene was 100%. Finally, 175 mmol of methanol was added as a polymerization terminator, and mixed to deactivate all of the active terminals of the triblock chains to form a styrene-isoprene-styrene triblock copolymer A1. A portion of the resulting reaction solution was retrieved, and the block copolymer was evaluated according to the measurement methods describe above. The results are shown in Table 2.

As the polymerization step proceeded, the viscosity of the solution increased. Thus, when the stirring was continued under the same rotation conditions, the Reynolds number for stirring decreased in later steps. However, even in the stage before the final addition of the polymerization terminator where the viscosity became the highest, still the Reynolds number for stirring maintained ≥ 500. The power used for stirring was not largely changed when the viscosity of the solution changed, and was still ≥ 2.5 kW/m$^3$ (the same applies to Production Examples 2 to 7).

In the styrene and isoprene used as the monomers, the amounts of impurities such as water were controlled, and the amount of deactivation components, such as water, which deactivate the polymerization initiator was $1 \times 10^{-3}$ mol/kg or less.

To 100 parts of the reaction solution obtained as described above (containing 30 parts of the polymer component), 0.3 parts of 2,6-di-tert-butyl-p-cresol was added as an antioxidant and mixed, and the mixed solution was added dropwise into hot water heated to 85 to 95°C in small portions to volatilize the solvent, obtaining a precipitate. The precipitate was pulverized and hot air dried at 85°C. Thereby, a mixture containing the block copolymer A1 and the block copolymer B1 was recovered.

The weight ratio of the block copolymer A1 and the block copolymer B1 in the above mixture (block copolymer Al/block copolymer B1) was 57/43.

[Production Examples 2 to 5]

Reaction solutions and mixtures were obtained in the same manner as in Production Example 1 except that the amounts of raw materials such as styrene and isoprene used were changed as shown in Table 1. The evaluation results are shown in Table 2.

[Production Example 6]

A reaction solution and a mixture were obtained in the same manner as in Production Example 1 except that the amounts of raw materials such as styrene and isoprene used were changed as shown in Table 1. The evaluation results are shown in Table 2.

[Production Example 7]

A reaction solution and a mixture were obtained in the same manner as in Production Example 1 except that the amounts of raw materials such as styrene and isoprene were changed as shown in Table 1, and the fourth polymerization stage was not carried out. The evaluation results are shown in Table 2.

[Table 1]

Table 1

| | | Production Examples | | | | | | |
|---|---|---|---|---|---|---|---|---|
| | | 1 | 2 | 3 | 4 | 5 | 6 | 7 |
| Cyclohexane (kg) | | 23.3 | 23.3 | 23.3 | 23.3 | 23.3 | 23.3 | 23.3 |
| TMEDA(mmol) | | 2.6 | 3.8 | 8.5 | 11.1 | 19.4 | 2.7 | 6.3 |
| n-Butyllithium (mmol) | | 87.3 | 76.9 | 85.3 | 92.8 | 102.2 | 54.2 | 125.0 |
| Styrene (kg) | [First polymerization stage] | 0.91 | 0.92 | 0.97 | 0.94 | 1.05 | 0.49 | 1.45 |
| Isoprene (kg) | [Second polymerization stage] | 5.2 | 5.0 | 5.0 | 6.3 | 6.0 | 3.3 | 7.1 |
| Styrene (kg) | [Third polymerization stage] | 0.91 | 0.92 | 0.97 | 0.94 | 1.05 | 0.49 | 1.45 |
| Methanol (mmol) | [After third polymerization stage] | 53.7 | 38.5 | 64 | 62.2 | 55.2 | 43.3 | 250 |
| Styrene (kg) | [Fourth polymerization stage] | 2.97 | 3.15 | 3.06 | 1.82 | 1.89 | 5.7 | - |
| Methanol (mmol) | [After fourth polymerization stage] | 175 | 154 | 171 | 186 | 204 | 108 | - |

[Table 2]

Table 2

| | Production Example 1 | Production Example 2 | Production Example 3 | Production Example 4 | Production Example 5 | Production Example 6 | Production Example 7 |
|---|---|---|---|---|---|---|---|
| Block copolymer A | A1 | A2 | A3 | A4 | A5 | A6 | |
| Weight average molecular weight ($\times 10^3$) | 212 | 216 | 276 | 189 | 155 | 715 | |
| Molecular weight distribution (Mw/Mn) | 1.11 | 1.09 | 1.14 | 1.04 | 1.08 | 1.22 | |

(continued)

| | Production Example 1 | Production Example 2 | Production Example 3 | Production Example 4 | Production Example 5 | Production Example 6 | Production Example 7 |
|---|---|---|---|---|---|---|---|
| Weight average molecular weight of Ar1$^a$ ($\times 10^3$) | 10 | 12 | 11 | 10 | 10 | 10 | |
| Weight average molecular weight of Ar2$^a$ ($\times 10^3$) | 114 | 108 | 178 | 80 | 58 | 606 | |
| Weight average molecular weight of D$^a$ ($\times 10^3$) | as | 96 | 87 | 99 | 86 | 89 | |
| Ratio of Cis/Trans structures of D$^a$ (mol%) | 2.5 | 2.4 | 2.4 | 2.3 | 2.1 | 2.4 | |
| Styrene unit content (wt%) | 65 | 62 | 79 | 54 | 51 | 90 | |
| Block copolymer B | B1 | B2 | B3 | B4 | B5 | B6 | B7 |
| Weight average molecular weight ($\times 10^3$) | 110 | 122 | 111 | 121 | 108 | 108 | 120 |
| Molecular weight distribution (Mw/Mn) | 1.05 | 1.05 | 1.03 | 1.02 | 1.06 | 1.01 | 1.01 |
| Weight average molecular weight of Ar1$^b$ ($\times 10^3$) | 10 | 12 | 11 | 10 | 10 | 10 | 12 |
| Weight average molecular weight of Ar2$^b$ ($\times 10^3$) | 10 | 12 | 11 | 10 | 10 | 10 | 12 |
| Weight average molecular weight of D$^b$ ($\times 10^3$) | as | 96 | 87 | 121 | 86 | 89 | 96 |

(continued)

| | Production Example 1 | Production Example 2 | Production Example 3 | Production Example 4 | Production Example 5 | Production Example 6 | Production Example 7 |
|---|---|---|---|---|---|---|---|
| Ratio of Cis/Trans structures of D[b] (mol%) | 2.5 | 2.4 | 2.4 | 2.3 | 2.1 | 2.4 | 2.5 |
| Styrene unit content (wt%) | 26 | 27 | 28 | 23 | 26 | 23 | 29 |
| Weight ratio A/B | 57/43 | 66/34 | 48/52 | 45/55 | 56/44 | 66/34 | 0/100 |
| Total styrene content | 48 | 50 | 50 | 37 | 40 | 67 | 29 |

[Production Example 8]

To a pressure-resistant reactor, 23.3 kg of cyclohexane, 6.7 mmol of TMEDA, and 0.4 kg of styrene were added and stirred with a Reynolds number for stirring of 160,000 and a power used for rotating the stirring blade of 3 kW/m$^3$ at 40°C. To the mixture, 66.7 mmol of n-butyllithium was added while the stirring was continued, and polymerization was carried out for 1 hour with heating to 50°C and the stirring was continued under the same rotation conditions. The polymerization conversion of styrene was 100%. Subsequently, while the temperature was controlled to maintain 50 to 60°C and the stirring was continued under the same rotation conditions, 9.2 kg of isoprene was continuously added to the reactor over 1 hour. After the addition of the isoprene was completed, the polymerization was continued for another 1 hour with stirring continued under the same rotation conditions. The polymerization conversion of isoprene was 100%. Next, while the temperature was controlled to maintain 50 to 60°C and the stirring was continued under the same rotation conditions, 0.4 kg of styrene was continuously added over 1 hour. After the addition of the styrene was completed, the polymerization was continued for another 1 hour with stirring continued under the same rotation conditions to form triblock chains having active terminals. The polymerization conversion of styrene was 100%. Finally, 133 mmol of methanol was added as a polymerization terminator and mixed to deactivate part of the active terminals of the triblock chains having active terminals to form a styrene-isoprene-styrene triblock copolymer C1. A portion of the resulting reaction solution was retrieved, and the block copolymer was evaluated according to the measurement methods describe above. The results are shown in Table 3.

As the polymerization step proceeded, the viscosity of the solution increased. Thus, when the stirring was continued under the same rotation conditions, the Reynolds number for stirring decreased in later steps. However, even in the stage before the final addition of the polymerization terminator where the viscosity became the highest, still the Reynolds number for stirring maintained ≥ 500. The power used for stirring was not largely changed when the viscosity of the solution changed, and was still ≥ 2.5 kW/m$^3$ (the same applies to Production Example 9) .

In the styrene and isoprene used as the monomers, the amounts of impurities such as water were controlled, and the amount of deactivation components, such as water, which deactivate the polymerization initiator was $1 \times 10^{-3}$ mol/kg or less.

To 100 parts of the reaction solution obtained as described above (containing 30 parts of the polymer component), 0.3 parts of 2,6-di-tert-butyl-p-cresol was added as an antioxidant and mixed, and the mixed solution was added dropwise into hot water heated to 85 to 95°C in small portions to volatilize the solvent, obtaining a precipitate. The precipitate was pulverized and hot air dried at 85°C. Thereby, a block copolymer C1 was recovered.

[Production Example 9]

A reaction solution and a block copolymer were obtained in the same manner as in Production Example 8 except that the amounts of raw materials such as styrene and isoprene used were changed as shown in Table 3. The evaluation results are shown in Table 4.

[Table 3]

Table 3

| | | Production Examples | |
|---|---|---|---|
| | | 8 | 9 |
| Cyclohexane (kg) | | 23.3 | 23.3 |
| TMEDA(mmol) | | 6.7 | 3.8 |
| n-Butyllithium (mmol) | | 66.7 | 76.9 |
| Styrene (kg) | [First polymerization stage] | 0.4 | 0.75 |
| Isoprene (kg) | [Second polymerization stage] | 9.2 | 8.5 |
| Styrene (kg) | [Third polymerization stage] | 0.4 | 0.75 |
| Methanol (mmol) | [After third polymerization stage] | 133 | 154 |

[Table 4]

Table 4

| | Production Example 8 | Production Example 9 |
|---|---|---|
| Block copolymer C | C1 | C2 |
| Weight average molecular weight ($\times 10^3$) | 225 | 195 |
| Molecular weight distribution (Mw/Mn) | 1.13 | 1.05 |
| Weight average molecular weight of Ar1$^c$ ($\times 10^3$) | 6 | 10 |
| Weight average molecular weight of Ar2$^c$ ($\times 10^3$) | 6 | 10 |
| Weight average molecular weight of D$^c$ ($\times 10^3$) | 213 | 176 |
| Ratio of Cis/Trans structures of D$^c$ (mol%) | 2.3 | 2.4 |
| Styrene unit content (wt%) | 8 | 15 |

[Production Example 10]

To a pressure-resistant reactor, 23.3 kg of cyclohexane, 5.9 mmol of TMEDA, and 1.00 kg of styrene were added and stirred with a Reynolds number for stirring of 160,000 and a power used for rotating the stirring blade of 3 kW/m$^3$ at 40°C. To the mixture, 117.6 mmol of n-butyllithium was added while the stirring was continued, and polymerization was carried out for 1 hour with heating to 50°C and the stirring was continued under the same rotation conditions. The polymerization conversion of styrene was 100%. Subsequently, while the temperature was controlled to maintain 50 to 60°C and the stirring was continued under the same rotation conditions, 9.00 kg of isoprene was continuously added to the reactor over 1 hour. After the addition of the isoprene was completed, the polymerization was continued for another 1 hour with stirring continued under the same rotation conditions to form diblock chains having active terminals. The polymerization conversion of isoprene was 100%. Next, 23.5 mmol of dimethyldichlorosilane was added as a coupling agent with stirring continued under the same rotation conditions to perform a coupling reaction for 2 hours to form a styrene-isoprene coupling block copolymer D1. Subsequently, 235 mmol of methanol was added as a polymerization terminator and mixed to deactivate all of the active terminals of the diblock chains to form a styrene-isoprene diblock copolymer E1. A portion of the resulting reaction solution was retrieved, and the block copolymer was evaluated according to the measurement methods describe above. The results are shown in Table 6.

As the polymerization step proceeded, the viscosity of the solution increased. Thus, when the stirring was continued under the same rotation conditions, the Reynolds number for stirring decreased in later steps. However, even in the stage before the final addition of the polymerization terminator where the viscosity became the highest, still the Reynolds number for stirring maintained $\geq 500$. The power used for stirring was not largely changed when the viscosity of the solution changed, and was still $\geq 2.5$ kW/m$^3$.

In the styrene and isoprene used as the monomers, the amounts of impurities such as water were controlled, and the amount of deactivation components, such as water, which deactivate the polymerization initiator was $1 \times 10^{-3}$ mol/kg or less.

To 100 parts of the reaction solution obtained as described above, 0.3 parts of 2,6-di-tert-butyl-p-cresol was added as an antioxidant and mixed, and the mixed solution was added dropwise into hot water heated to 85 to 95°C in small portions to volatilize the solvent, obtaining a precipitate. The precipitate was pulverized and hot air dried at 85°C. Thereby, a mixture containing the block copolymer D1 and the block copolymer E1 was recovered.

The weight ratio of the block copolymer D1 and the block copolymer E1 in the above mixture (block copolymer D1/block copolymer E1) was 40/60.

[Table 5]

Table 5

|  |  | Production Example |
| --- | --- | --- |
|  |  | 10 |
| Cyclohexane (kg) |  | 23.3 |
| TMEDA (mmol) |  | 5.9 |
| n-Butyllithium (mmol) |  | 117.6 |
| Styrene (kg) | [First polymerization stage] | 100 |
| Isoprene (kg) | [Second polymerization stage] | 9.00 |
| Dimethyldichlorosilane (mmol) | [After second polymerization stage] | 23.5 |
| Methanol (mmol) | [After second polymerization stage] | 235 |

[Table 6]

Table 6

|  | Production Example 10 |
| --- | --- |
| Block copolymer D | D1 |
| Weight average molecular weight ($\times 10^3$) | 247 |
| Molecular weight distribution (Mw/Mn) | 1.02 |
| Weight average molecular weight of Ar$^d$ ($\times 10^3$) | 9 |
| Weight average molecular weight of D$^d$ ($\times 10^3$) | 115 |
| Ratio of Cis/Trans structures of D$^d$ (mol%) | 2.5 |
| Styrene unit content (wt%) | 10 |
| Number of branches n | 2 |
| Block copolymer E | E1 |
| Weight average molecular weight ($\times 10^3$) | 123 |
| Molecular weight distribution (Mw/Mn) Each number of branches | 1.02 |
| Weight average molecular weight of Ar$^e$ ($\times 10^3$) | 9 |
| Weight average molecular weight of D$^e$ ($\times 10^3$) | 115 |
| Ratio of Cis/Trans structures of D$^e$ (mol%) | 2.5 |

(continued)

| | Production Example 10 |
|---|---|
| Styrene unit content (wt%) | 10 |
| Weight ratio D/E | 40/60 |

[Example 1]

The mixture of the block copolymer A1 and the block copolymer B1 obtained in Production Example 1, and the block copolymer C1 obtained in Production Example 8 were mixed such that the weight ratios of the block copolymers were as shown in Table 7 to prepare a composition. The resulting composition was fed into a twin screw extruder equipped with a T-die. Subsequently, the composition was heated and melted at 200°C, continuously kneaded and extruded for 20 minutes onto a cooling roll to be allowed to cool, and then wound by a winding roll to form a film having an average thickness of 0.05 mm. The film according to Example 1 thus obtained was subjected to the measurements and the evaluations for the ratio ($M_{MD}/M_{TD}$) between the stress at 100% elongation in the MD direction ($M_{MD}$) and the stress at 100% elongation in the TD direction ($M_{TD}$), the yield point elongation in the MD direction, the stress at 200% elongation in the MD direction, the permanent tension set in the MD direction, and the film width according to the methods described above. On the other hand, a 2-mm sheet was separately produced, and subjected to the measurements for the storage modulus and the loss tangent ($\tan\delta$). The results are shown in Table 7. The conditions for forming the film are detailed below.

(Forming conditions for films)

Compound processing rate: 5 kg/hour
Film take-off speed: 4 m/minute
Extruder temperature: adjusted to inlet of 100°C and T-die of 200°C
Screw: full flight
Extruder L/D: 30
T-Die: 200 mm wide, 0.5 mm lip
Distance between tip of T-die and cooling roll: 2 mm
Cooling roll temperature: 23°C

[Examples 2 to 6, Comparative Examples 1 to 6]

Compositions and films were obtained in the same manner as in Example 1 except that the weight ratio of each block copolymer was changed as shown in Table 7, and were evaluated in the same manner. The results are shown in Table 7.

[Comparative Example 7]

A film was obtained in the same manner as in Example 1 except that a polyolefin elastomer F1 (trade name: Vistamaxx 3000 (available from Exxon Mobil Corporation), an ethylene-propylene block copolymer, ethylene content: 11% by weight, hardness (Shore A): 82, Vicat softening point: 66°C) was used instead of the block copolymers, and was evaluated in the same manner. The results are shown in Table 7.

[Comparative Example 8]

A film was obtained in the same manner as in Example 1 except that a polyolefin elastomer F2 (trade name: Vistamaxx 3020 (available from Exxon Mobil Corporation), ethylene content: 11% by weight, hardness (Shore A): 84, Vicat softening point: 68°C) was used instead of the block copolymers, and was evaluated in the same manner. The results are shown in Table 7.

[Table 7]

Table 7

| | | Styrene unit content (wt%) | Examples (wt%) | | | | | | Comparative Examples (wt%) | | | | | | | |
|---|---|---|---|---|---|---|---|---|---|---|---|---|---|---|---|---|
| | | | 1 | 2 | 3 | 4 | 5 | 6 | 1 | 2 | 3 | 4 | 5 | 6 | 7 | 8 |
| A1 | SIS | 65 | 37 | | | | | | | 37 | | | | 28.5 | | |
| A2 | SIS | 62 | | 33 | | | | | | | | | | | | |
| A3 | SIS | 79 | | | 36 | | | | | | | | | | | |
| A4 | SIS | 54 | | | | 22.5 | | 16 | | | | | | | | |
| A5 | SIS | 51 | | | | | 28 | | | | | | | | | |
| A6 | SIS | 90 | | | | | | | 50 | | | | | | | |
| B1 | SIS | 26 | 28 | | | | | | | 28 | | | | 21.5 | | |
| B2 | SIS | 27 | | 17 | | | | | | | | | | | | |
| B3 | SIS | 28 | | | 39 | | | | | | | | | | | |
| B4 | SIS | 23 | | | | 27.5 | | 19 | | | | | | | | |
| B5 | SIS | 26 | | | | | 22 | | | | | | | | | |
| B6 | SIS | 23 | | | | | | | 25 | | | | | | | |
| B7 | SIS | 29 | | | | | | | | | 100 | | 50 | | | |
| C1 | SIS | 8 | | | | | | | | 35 | | | | | | |
| C2 | SIS | 15 | 35 | 50 | 25 | 50 | 50 | 65 | 25 | | | 100 | 50 | | | |
| D1 | SIS | 10 | | | | | | | | | | | | 20 | | |
| E1 | SIS | 10 | | | | | | | | | | | | 30 | | |
| F1 | POE | - | | | | | | | | | | | | | 100 | |
| F2 | POE | - | | | | | | | | | | | | | | 100 |

EP 4 491 668 A1

| | | Styrene unit content (wt%) | Examples (wt%) | | | | | | Comparative Examples (wt%) | | | | | | | |
|---|---|---|---|---|---|---|---|---|---|---|---|---|---|---|---|---|
| | | | 1 | 2 | 3 | 4 | 5 | 6 | 1 | 2 | 3 | 4 | 5 | 6 | 7 | 8 |
| Physical properties of composition | Styrene content [%] | | 37 | 33 | 43 | 26 | 28 | 23 | 54 | 34 | 29 | 15 | 22 | 29 | - | - |
| | Total weight average molecular weight ($\times 10^{-3}$) | | 178 | 190 | 191 | 173 | 165 | 180 | 430 | 188 | 120 | 195 | 158 | 170 | 300 | 310 |
| | Storage modulus @23°C [MPa] | | 1.6 | 1.2 | 2.2 | 0.7 | 1.0 | 0.5 | 14.0 | 1.6 | 1.0 | 0.3 | 0.6 | 0.5 | 8.5 | 8.6 |
| | Tan$\delta$ @23°C [-] | | 0.07 | 0.08 | 0.10 | 0.05 | 0.13 | 0.04 | 0.13 | 0.21 | 0.05 | 0.06 | 0.06 | 0.23 | 0.06 | 0.06 |
| | Ratio of stresses at 100% ($M_{MD}/M_{TD}$) [-] | | 1.2 | 1.1 | 1.8 | 1.1 | 1.9 | 1.1 | 1.9 | 1.2 | 3.5 | 1.0 | 2.5 | 1.1 | 1.1 | 10 |
| | Yield point elongation (MD direction) | | >200 | >200 | >200 | >200 | >200 | >200 | 4 | >200 | 8 | >200 | >200 | >200 | 35 | 40 |
| Results of evaluations | Stress at 200% (MD direction) | | ○ | ○ | ○ | ○ | ○ | ○ | ○ | ○ | ○ | × | ○ | ○ | ○ | ○ |
| | Permanent tension set (MD direction) | | ○ | ○ | ○ | ○ | ○ | ○ | × | × | × | ○ | ○ | × | × | × |
| | Film width | | ○ | ○ | ○ | ○ | ○ | ○ | ○ | ○ | × | ○ | × | ○ | ○ | ○ |

As shown in Table 7, by using the block copolymer compositions that satisfied all of the following (1) to (4), good results were obtained for all of the stress at 200% elongation in the MD direction, the permanent tension set in the MD direction, and the film width, and film-shaped elastic bodies producing a high stress and having a high stretchability and a large width were provided (Examples 1 to 6).

(1) The storage modulus at 23°C is more than 0.3 MPa.
(2) The loss tangent (tan$\delta$) at 23°C is 0.20 or less.
(3) When the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5.
(4) When the thermoplastic elastomer is extruded into a film shape by melt extrusion using a melt extrusion apparatus, and cooled and solidified to form a film and the film is stretched in the MD direction, the film does not have a yield point at an elongation rate of 200% or less.

On the other hand, when the storage modulus as described in (1) above was too low, the stress became low (Comparative Example 4). When (2) above was not satisfied and when (4) above was not satisfied, the permanent tension set became large (Comparative Examples 1 to 3, and 6 to 8). When (3) above was not satisfied, the change in film width became large (Comparative Examples 3 and 5).

[Example 7]

A composition and a film were obtained in the same manner as in Example 1 except that the mixture of the block copolymer A4 and the block copolymer B4 obtained in Production Example 4, the block copolymer C2 obtained in Production Example 9, and a polyolefin elastomer F1 (trade name: Vistamaxx 3000 (available from Exxon Mobil Corporation), an ethylene-propylene block copolymer, ethylene content: 11% by weight, hardness (Shore A): 82, Vicat softening point: 66°C) were mixed such that the weight ratios of the block copolymers were as shown in Table 8, and was evaluated in the same manner. The results are shown in Table 8.

In Example 7, the property of loss of resilience was measured according to the following method. The resulting film was stretched up to 100% in the MD direction, and held for 6 hours in that state. After the film was held for 6 hours, the film was allowed to recover the original state. The change in size during the 6-hour holding was calculated according to the following equation, "change in size (%) = {(length in MD direction after 6-hour holding at 100% elongation) - (length in MD direction before test)}/(length in MD direction before test} $\times$ 100", and was evaluated based on the following criteria.

○: The change in size is less than 30%
×: The change in size is 30% or more

[Examples 8 and 9, and Comparative Example 1]

Compositions and films were obtained in the same manner as in Example 7 except that the amounts of the mixture of the block copolymer A4 and the block copolymer B4 obtained in Production Example 4, the block copolymer C2 obtained in Production Example 9, and the polyolefin elastomer F1 were changed as shown in Table 8, and were evaluated in the same manner. The results are shown in Table 8.

[Table 8]

Table 8

| | | Styrene unit content (wt%) | Examples (wt%) | | | Comparative Example (wt%) |
|---|---|---|---|---|---|---|
| | | | 7 | 8 | 9 | 9 |
| A1 | SIS | 65 | | | | |
| A2 | SIS | 62 | | | | |
| A3 | SIS | 79 | | | | |
| A4 | SIS | 54 | 21 | 20 | 16 | 9 |

(continued)

| | | Styrene unit content (wt%) | Examples (wt%) | | | Comparative Example (wt%) |
|---|---|---|---|---|---|---|
| | | | 7 | 8 | 9 | 9 |
| A5 | SIS | 51 | | | | |
| A6 | SIS | 90 | | | | |
| B1 | SIS | 26 | | | | |
| B2 | SIS | 27 | | | | |
| B3 | SIS | 28 | | | | |
| B4 | SIS | 23 | 26 | 25 | 19 | 11 |
| B5 | SIS | 26 | | | | |
| B6 | SIS | 23 | | | | |
| B7 | SIS | 29 | | | | |
| C1 | SIS | 8 | | | | |
| C2 | SIS | 15 | 48 | 45 | 35 | 20 |
| D1 | SIS | 10 | | | | |
| E1 | SIS | 10 | | | | |
| F1 | POE | - | 5 | 10 | 30 | 60 |
| F2 | POE | - | | | | |
| Physical properties of composition | Styrene content [%] | | 25 | 23 | 18 | 10 |
| | Total weight average molecular weight ($\times 10^{-3}$) | | 180 | 186 | 211 | 249 |
| | Storage modulus @23°C [MPa] | | 1.5 | 2.0 | 4.9 | 8.4 |
| | Tan$\delta$ @23°C [-] | | 0.05 | 0.05 | 0.05 | 0.06 |
| | Ratio of stresses at 100% ($M_{MD}/M_{TD}$) [-] | | >200 | >200 | >200 | 1100 |
| | Yield point elongation (MD direction) | | 1.1 | 1.1 | 1.1 | 1.1 |
| Results of evaluations | Stress at 200% (MD direction) | | ○ | ○ | ○ | ○ |
| | Permanent tension set (MD direction) | | ○ | ○ | ○ | × |
| | Film width | | ○ | ○ | ○ | ○ |
| | Property of loss of resilience (porous film) | | ○ | ○ | ○ | ○ |

As shown in Table 8, by using the block copolymer compositions that satisfied all of the above-described (1) to (4), and contained an aromatic vinyl block copolymer in a proportion of more than 50% by weight, and contained a polyolefin elastomer in a proportion of more than 1% by weight and less than 50% by weight, good results were obtained for all of the stress at 200% elongation in the MD direction, the permanent tension set in the MD direction, and the film width, and film-shaped elastic bodies producing a high stress and having a high stretchability and a large width were provided. Furthermore, the films had an excellent property of loss of resilience (stress relaxation property), and even when the films were used as a porous film, problems of tearing and loss of resilience were effectively suppressed (Examples 7 to 9).

## Claims

1.  A thermoplastic elastomer,

    wherein the storage modulus at 23°C is more than 0.3 MPa;
    the loss tangent (tan$\delta$) at 23°C is less than 0.20;
    when the thermoplastic elastomer is extruded into a film shape by melt extrusion using a melt extrusion apparatus, and cooled and solidified to form a film, the ratio ($M_{MD}/M_{TD}$) between the stress at 100% elongation in the MD direction ($M_{MD}$) and the stress at 100% elongation in the TD direction ($M_{TD}$) of the film is less than 2.5; and when the film is stretched in the MD direction, the film does not have a yield point at an elongation rate of 200% or less.

2. The thermoplastic elastomer according to claim 1, containing an aromatic vinyl block copolymer.

3. The thermoplastic elastomer according to claim 2, wherein the aromatic vinyl block copolymer is an aromatic vinyl-conjugated diene block copolymer.

4. The thermoplastic elastomer according to claim 2 or 3, containing the aromatic vinyl block copolymer in a proportion of more than 50% by weight, and a polyolefin elastomer in a proportion of more than 1% by weight and less than 50% by weight.

5. The thermoplastic elastomer according to claim 4, wherein the polyolefin elastomer contains an ethylene unit.

6. The thermoplastic elastomer according to any one of claims 1 to 5, wherein the thermoplastic elastomer is for use in a stretchable member which is stretched and contracted in the MD direction.

7. The thermoplastic elastomer according to any one of claims 1 to 6, wherein the thermoplastic elastomer is used as a stretchable member by extruded into a film shape by melt extrusion, and successively laminated and welded onto a substrate sheet.

8. An elastic body obtained by using the thermoplastic elastomer according to any one of claims 1 to 5.

9. A film obtained by using the thermoplastic elastomer according to any one of claims 1 to 5.

10. The elastic body or the film according to claim 8 or 9 used in applications where the elastic body or the film is stretched and contracted in the MD direction.

11. A porous film obtained by using the thermoplastic elastomer according to claim 4 or 5.

12. A stretchable member obtained by using the thermoplastic elastomer according to any one of claims 1 to 7.

FIG. 1

# INTERNATIONAL SEARCH REPORT

| International application No. |
|---|
| **PCT/JP2023/007889** |

**A. CLASSIFICATION OF SUBJECT MATTER**

*C08L 53/02*(2006.01)i
FI: C08L53/02

According to International Patent Classification (IPC) or to both national classification and IPC

**B. FIELDS SEARCHED**

Minimum documentation searched (classification system followed by classification symbols)

C08L53/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2020/045496 A1 (ZEON CORP) 05 March 2020 (2020-03-05) <br> entire text | 1-12 |
| A | WO 2016/002764 A1 (ASAHI KASEI CHEMICALS CORP ) 07 January 2016 (2016-01-07) <br> entire text | 1-12 |
| A | JP 2008-7654 A (ZEON CORP) 17 January 2008 (2008-01-17) <br> entire text | 1-12 |
| A | JP 2012-77158 A (ZEON CORP) 19 April 2012 (2012-04-19) <br> entire text | 1-12 |
| A | JP 9-279460 A (IDEMITSU PETROCHEM CO LTD) 28 October 1997 (1997-10-28) <br> entire text | 1-12 |

☐ Further documents are listed in the continuation of Box C.    ☑ See patent family annex.

| | |
|---|---|
| \* Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **09 May 2023** | **23 May 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

INTERNATIONAL SEARCH REPORT
Information on patent family members

International application No.

**PCT/JP2023/007889**

| Patent document cited in search report | | | Publication date (day/month/year) | Patent family member(s) | | | Publication date (day/month/year) |
|---|---|---|---|---|---|---|---|
| WO | 2020/045496 | A1 | 05 March 2020 | EP | 3845599 | A1 | |
| | | | | entire text | | | |
| WO | 2016/002764 | A1 | 07 January 2016 | US | 2017/0137618 | A1 | |
| | | | | entire text | | | |
| JP | 2008-7654 | A | 17 January 2008 | (Family: none) | | | |
| JP | 2012-77158 | A | 19 April 2012 | (Family: none) | | | |
| JP | 9-279460 | A | 28 October 1997 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020045496 A

**Non-patent literature cited in the description**

- *Rubber Chem. Technol*, 1972, vol. 45, 1295